# EUROPÄISCHE PATENTSCHRIFT

(11) **EP 2 079 707 B1**
(45) Veröffentlichungstag und Bekanntmachung des Hinweises auf die Patenterteilung: **03.12.2014**
(21) Anmeldenummer: 07821055.6
(22) Anmeldetag: 09.10.2007
(51) Int. Cl.: C07D 233/58

(54) **VERFAHREN ZUR UMSETZUNG VON 1,3-HETERO-AROMATISCHEN 2-CARBOXYLATEN MIT WASSER**
METHOD FOR REACTING 1,3-HETEROAROMATIC 2-CARBOXYLATES WITH WATER
PROCÉDÉ DE MISE EN RÉACTION DE 2-CARBOXYLATES 1,3 HÉTÉROAROMATIQUES AVEC DE L'EAU

(30) Priorität: 10.10.2006 AT 16862006
(43) Veröffentlichungstag der Anmeldung: 22.07.2009
(73) Patentinhaber: proionic GmbH & Co KG, 8074 Grambach (AT)
(72) Erfinder: KALB, Roland, A-8700 Leoben (AT)
(74) Vertreter: Vinazzer, Edith
(86) Internationale Anmeldenummer: PCT/EP2007/060684
(87) Internationale Veröffentlichungsnummer: WO 2008/052863

(56) Entgegenhaltungen:
- WO-A-01/77081
- WO-A-2005/021484
- WO-A-2006/009630
- WO-A-2006/027069
- GB-A- 2 378 701
- SARBU T ET AL: "ATRP OF METHYL METHACRYLATE IN THE PRESENCE OF IONIC LIQUIDS WITH FERROUS AND CUPROUS ANIONS" MACROMOLECULAR CHEMISTRY AND PHYSICS, WILEY-VCH VERLAG, WEINHEIM, DE, Bd. 202, Nr. 17, 11. Dezember 2001 (2001-12-11), Seiten 3379-3391, XP001125287 ISSN: 1022-1352
- CASTELLS J ET AL: "Use of thiazolium-2-carboxylates to induce benzoin condensations" TETRAHEDRON LETTERS, ELSEVIER, AMSTERDAM, Bd. 26, Nr. 44, 1. Januar 1985 (1985-01-01), Seiten 5457-5458, XP002247673 ISSN: 0040-4039
- HOLBREY J D ET AL: "1,3-Dimethylimidazolium-2-carboxylate: The unexpected synthesis of an ionic liquid precursor and carbene-CO2 adduct" CHEMICAL COMMUNICATIONS 2003 GB, Nr. 1, 2003, Seiten 28-29, XP002484031 ISSN: 1359-7345
- TOMMASI I ET AL: "Utilisation of 1,3-dialkylimidazolium-2-carboxylates as CO2-carriers in the presence of Na<+> and K<+>: application in the synthesis of carboxylates, monomethylcarbonate anions and halogen-free ionic liquids" TETRAHEDRON LETTERS, ELSEVIER, AMSTERDAM, NL, Bd. 46, Nr. 12, 21. März 2005 (2005-03-21), Seiten 2141-2145, XP004768425 ISSN: 0040-4039
- BRIDGES, NICHOLAS J. ET AL: "An intermediate for the clean synthesis of ionic liquids: isolation and crystal structure of 1,3-dimethylimidazolium hydrogen carbonate monohydrate" CHEMISTRY--A EUROPEAN JOURNAL , 13(18), 5207-5212 CODEN: CEUJED; ISSN: 0947-6539, 2007, XP002468203
- SMIGLAK, MARCIN ET AL: "Ionic liquids via reaction of the zwitterionic 1,3-dimethylimidazolium-2- carboxylate with protic acids. Overcoming synthetic limitations and establishing new halide free protocols for the formation of ILs" GREEN CHEMISTRY , 9(1), 90-98 CODEN: GRCHFJ; ISSN: 1463-9262, 2007, XP008088232
- L. NYULÁSZI ET AL: "Investigation of heterocyclic compounds containing a P=C or As=C bond by ultraviolet photoelectron spectroscopy", JOURNAL OF ORGANOMETALLIC CHEMISTRY, vol. 373, no. 1, 1 September 1989 (1989-09-01), pages 49-55, ISSN: 0022-328X, DOI: 10.1016/0022-328X(89)85023-5
- DUPRÉ DONALD B ET AL: "Topological analysis of the electron density in model azolium systems for thiamin structure-function: sulfur is the electron sink and positively polarized carbanions act as nucleophiles.", THE JOURNAL OF PHYSICAL CHEMISTRY. A 25 AUG 2005, vol. 109, no. 33, 25 August 2005 (2005-08-25), pages 7606-7612, ISSN: 1089-5639
- HAAKE P ET AL: "Fundamental studies on models for thiamine. Generation of ylides of oxazolium, imidazolium, and thiazolium ions by decarboxylation. Applications to the structure of the thiamine ylide.", JOURNAL OF THE AMERICAN CHEMICAL SOCIETY 15 DEC 1971, vol. 93, no. 25, 15 December 1971 (1971-12-15), pages 7045-7049, ISSN: 0002-7863
- Gilchrist T.L.: "Heterocyclenchemie", ISBN: 3-527-29223-3 pages 322-323,

## Beschreibung

Die Erfindung betrifft ein Verfahren zur Herstellung von 1,3-heteroaromatischen Hydrogencarbonaten und/oder 1,3-heteroaromatischen Carbonaten aus 1,3-heteroaromatischen 2-Carboxylaten.

Ionische Verbindungen - Verbindungen, welche ionische Flüssigkeiten oder ionische Feststoffe oder Gemische derselben sind - weisen äußerst interessante Eigenschaften auf, wie beispielsweise das Fehlen eines (meßbaren) Dampfdrucks (mit wenigen Ausnahmen: siehe Martyn J. Earle, Jose M.S.S. Esperanc, Manuela A. Gilea1, Jose N. Canongia Lopes, Luis P.N. Rebelo, Joseph W. Magee, Kenneth R. Seddon & Jason A. Widegren, Nature 2006, Vol.439, 831-834), einen sehr großen Liquidusbereich, gute elektrische Leitfähigkeit und ungewöhnliche Solvatations-Eigenschaften. Diese Eigenschaften prädestinieren sie für den Einsatz in verschiedenen Bereichen technischer Anwendungen. So können sie beispielsweise als Lösungsmittel (bei organischer und anorganischer Synthese im Allgemeinen, bei der Übergangsmetallkatalyse, der Biokatalyse, der PhasentransferKatalyse, bei Mehrphasen-Reaktionen, in der Photochemie, in der Polymersynthese und der Nanotechnologie), als Extraktionsmittel (bei der flüssig-flüssig- und der flüssig-gasförmig Extraktion im Allgemeinen, der Entschwefelung von Rohöl, der Entfernung von Schwermetallen aus Abwässern, der Flüssigmembranextraktion), als Elektrolyte (in Batterien, Brennstoffzellen, Kondensatoren, Solarzellen, Sensoren, in der Elektrochromie, der Galvanotechnik, in der elektrochemischen Metallbearbeitung, in der elektrochemischen Synthese im Allgemeinen, bei der elektroorganischen Synthese, der Nanotechnologie), als Schmierstoffe, als Thermofluide, als Gele, als Reagenzien zur organischen Synthese, in der "Green Chemistry" (Ersatz für Volatile Organic Compounds), als Antistatika, in Spezialanwendungen der Analytik (Gaschromatographie, Massenspektroskopie, Kapillarzonenelektrophorese), als Flüssigkristalle, etc. eingesetzt werden. Diesbezüglich wird beispielsweise auf "Rogers, Robin D.; Seddon, Kenneth R. (Eds.); lonic Liquids - Industrial Applications to Green Chemistry, ACS Symposium Series 818, 2002; ISBN 0841237891" und "Wasserscheid, Peter; Welton, Tom (Eds.); lonic Liquids in Synthesis, Verlag Wiley-VCH 2003; ISBN 3527305157" verwiesen.

Die Herstellung von 1,3-heteroaromatischen Carbonaten der Formel IVa (gemäß Anspruch 1) mit X = N, Y= N - R³, R¹ = Methyl, R³ = Butyl und R⁴ = R⁵ = H durch Umsetzung des entsprechenden Chloridsalzes mit Natriumcarbonat ist beispielsweise aus "ATRP of methyl methacrylate in the presence of ionic liquids with ferrous and cuprous anions", Sarbu T. et al, Macromolecular Chemistry and Physics, Bd. 202, Nr. 17, 2001, Seiten 3379-3391, XP00125287, bekannt.

Die Optimierung der Eigenschaften für die jeweilige Anwendung kann in weiten Grenzen durch eine Variation der Struktur von Anion und Kation bzw. eine Variation ihrer Kombination erfolgen, was den ionischen Flüssigkeiten die Bezeichnung "Designer Solvents" (siehe beispielsweise Freemantle, M.; Chem. Eng. News, 78, 2000, 37) eingebracht hat. In diesem Sinne wurden viele unsystematische Einzelsynthesen für die Herstellung ionischer Verbindungen im Labor- und Technikumsmaßstab entwickelt. Dementsprechend sind die Kosten der Produktion dieser Verbindungen extrem hoch und die Umsetzbarkeit im Industriemaßstab schwer realisierbar.

Ausgangsverbindungen der Synthesen solcher ionischer Verbindungen sind im Allgemeinen alkyl- oder arylsubstituierte organische Stickstoff-, Phosphor- oder Schwefelnucleophile (Amine, Phosphine, Sulfide, Heteroaromaten). Die derzeit bekannten Synthesemethoden werden am Beispiel der Synthese quaternärer Ammoniumverbindungen (siehe nachstehende Abbildung 1) hier kurz erläutert:

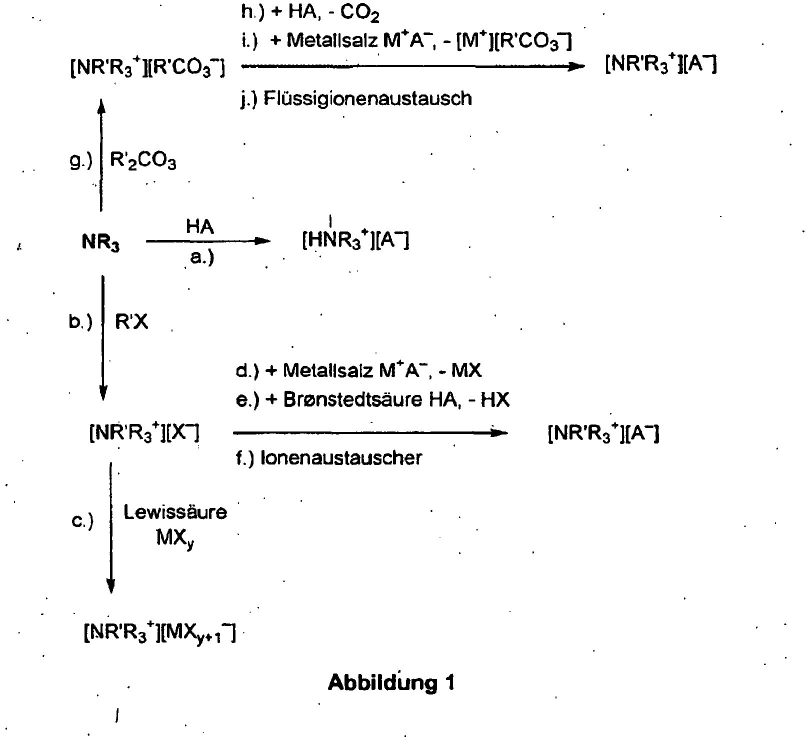

### a. Brønsted-Säure/Basenreaktion

Ein Amin NR₃ reagiert mit einer Protonensäure HA unter Bildung des Salzes [HNR₃⁺][A⁻]. Diese Darstellung ionischer Flüssigkeiten ist die älteste bekannte (Walden, P., Bull. Acad. Imper. Sci. (St. Petersburg), 1914, 1800) und führt beispielsweise bei der Reaktion von Ethylamin mit Salpetersäure zur Bildung von Ethylammoniumnitrat mit einem Schmelzpunkt von Fₚ = 13°C. In diesem Fall ist das Kation nicht quaterniert sondern nur protoniert. Diese organischen Salze kommen für typische Anwendungsgebiete ionischer Flüssigkeiten meist nicht in Frage, weil sie thermisch und chemisch instabil sind.

### b. Alkylierung bzw. Arylierung zu einem quaternären Salz

Das Amin R₃N wird mit einem Alkylierungs- oder Arylierungsreagens R'X in einer nucleophilen Substitution unter Bildung eines quaternierten Ammoniumsalzes [NR'R₃⁺][X⁻] umgesetzt (Wilkes, J.S.; Zaworotko, M.J., J. Chem. Soc., Chem. Commun., 13, 1992, 965). Als typische Alkylierungsmittel kommen dabei Halogenalkane wie z.B. 1-Chlorbutan, 1-Bromethan, Methyliodid oder Dialkylsulfate wie beispielsweise Dimethylsulfat oder Diethylsulfat zur Anwendung. Diese Alkylierungsmittel sind zwar reaktiv und bilden rasch das gewünschte Produkt, sie sind aber wie alle starken Alkylierungsreagenzien relativ. toxisch, zum Teil cancerogen und im Falle der Halogenalkane ggf. atmosphärenschädigend. Die gebildete ionische Verbindung [NR'R₃⁺][X⁻] kann wiederum selbst Ausgangsmaterial für weitere Umsetzungen gemäß den Reaktionen c - f sein. Sie ist im allgemeinen stark hygroskopisch:

### c. Reaktion mit einer Lewis-Säure

Zur Darstellung von ionischen Flüssigkeiten auf der Basis von Halogenmetallat-Anionen (siehe z.B. Wilkes, J.S.; Levisky, J.A.; Wilson, R.A.; Hussey, C.L., Inorg. Chem. 1982, 21, 1263) kann das quaternäre Ammoniumsalz [NR'R₃⁺][X⁻] direkt mit einer entsprechenden Lewis-Säure MX_{y} zur gewünschten Verbindung [NR'R₃⁺][MX_{y+1}⁻] umgesetzt werden. Im Falle des Tetrachloroaluminat-Anions [AlCl₄]⁻ wird z.B. ein entsprechendes Ammoniumchlorid [NR'R₃⁺][Cl⁻] direkt mit Aluminiumchlorid AlCl₃ versetzt.

Auf Halogenmetallat basierende ionische Flüssigkeiten sind extrem Wasser und Sauerstoff empfindlich, sehr korrosiv und werden nur für ganz spezielle Zwecke eingesetzt (beispielsweise Oleifin-Oligomerisierung, Friedel-Crafts Reaktionen). Bereits geringste Spuren Wasser führen zur Hydrolyse unter Bildung von HCl, HBr oder HI. Ihre Synthese gestaltet sich dementsprechend schwierig, nicht zuletzt auch aufgrund der Tatsache, daß das Ausgangsmaterial [NR'R₃⁺][X⁻] stark hygroskopisch ist.

### d. Anionenaustausch via Metathese

Hierbei wird das Anion eines quartären Ammoniumsalzes, das beispielsweise durch Alkylierung bzw. Arylierung gebildet wird, gegen das gewünschte Anion [A⁻] ausgetauscht. Hierzu wird das Salz [NR'R₃⁺][X⁻] in einem trockenen, organischen Lösungsmittel (z.B. Aceton, Acetonitril, Dichlormethan) mit einer stöchiometrischen Menge eines Metallsalzes (meist ein entsprechendes Alkali- oder Erdalkalisalz) [M⁺][A⁻] versetzt, welches das gewünschte Anion [A⁻] aufweist. Der Reaktionsansatz wird unter Ausschluß von Luftfeuchtigkeit typischerweise für einige Tage bis Wochen kräftig gerührt, wobei die gewünschte ionische Flüssigkeit in dem gewählten Lösungsmittel löslich ist und das Anion [X⁻] als unlösliches, festes Metallsalz [M⁺[X⁻] ausfällt und durch Filtration entfernt wird. Neben der sehr langen Reaktionszeit und den großen Mengen von Abfall [M⁺[X⁻] ist bei diesem Verfahren von Nachteil, daß man trockene Lösungsmittel verwenden muss, um einen kompletten Umsatz zu erzielen; ansonsten löst sich das zu entfernende Salz [M⁺][X⁻] in größeren Mengen im Lösungsmittel, wobei diese Halogenidspuren den späteren Einsatz der ionischen Flüssigkeit limitieren (Halogenide sind beispielsweise Katalysatorgifte; Entsorgung durch Verbrennen ist problematisch).

Das wasserfreie Arbeiten ist insbesondere im Industriemaßstab problematisch, da das Edukt [NR'R₃⁺][X⁻] im. Allgemeinen sehr hygroskopisch ist und der Produktionsprozess daher unter Ausschluss jeglicher Luftfeuchtigkeit erfolgen muss. Die genannten Nachteile könnten zwar durch den Einsatz von wässrigen Lösungsmittelsystemen und Silbersalzen [Ag⁺][A⁻] behoben werden (wobei die auch in Wasser sehr schwerlöslichen Silberhalogenide ausfallen), dies ist allerdings im Industriemaßstab aus ökonomischen Gründen völlig ausgeschlossen.

Ein kürzlich zum Patent angemeldetes einstufiges Produktionsverfahren (Wasserscheid, Peter.; Hilgers, Claus; Boesmann, Andreas; EP1182197 A1 (Solvent Innovation GmbH, Germany), 2002), welches die Reaktionen b und d vereint, kann zwar das Problem des Ausschlusses von Luftfeuchtigkeit zum Teil entschärfen (keine Isolation der hygroskopischen Zwischenstufe [NR'R₃⁺][X⁻] notwendig), allerdings bleiben die Reaktionszeiten nach wie vor äußerst lang, es fällt weiterhin Abfall an und die potentielle Gefahr der Verunreinigung des Endproduktes mit Halogenid-Ionen ist allgegenwärtig; darüber hinaus lassen sich Kondensationsprodukte und Verunreinigungen, die bei der Darstellung der hier nicht isolierten Zwischenstufe entstehen, nur nachträglich unter erheblich größerem Aufwand aus dem fertigen Produkt entfernen.

Allen Anionenaustauschverfahren via Metathese ist aber insbesondere gemeinsam, daß sie nicht allgemein anwendbar sind und nur für ganz bestimmte Kombinationen aus Kationen und Anionen zufriedenstellend funktionieren. Nur wenn die gewünschte ionische Flüssigkeit im Lösungsmittel ausreichend löslich ist (kann oft problematisch sein, zum Teil große Mengen Lösungsmittel nötig) und/oder das eingesetzte Metallsalz [M⁺][A⁻] im Lösungsmittel wesentlich besser löslich ist als das zu entfernende, kann mit Erfolg gerechnet werden. Des weiteren kann das ausgefällte Salz [M⁺][X⁻] zum Teil in der ionischen Flüssigkeit selbst löslich sein.

### e. Anionenaustausch via Brønsted-Säure

Eine weitere Variante des Anionenaustausches stellt die Reaktion von [NR'R₃⁺][X⁻] mit einer Brønstedsäure HA dar. In diesem Fall muss die zugehörige freie Säure des gewünschten Anions stärker sein als die entsprechende Säure HX, sodaß auch diese Reaktion nur für wenige Anionen [A⁻] in Frage kommt. Ist HX - wie im Falle der Halogenwasserstoffsäuren - eine flüchtige Säure, kann diese verhältnismäßig leicht im Vakuum entfernt werden (ionische Flüssigkeiten besitzen im Allgemeinen keinen messbaren Dampfdruck); ist die ionische Flüssigkeit hydrophob, kann die Säure HX mit Wasser extraktiv entfernt werden. In allen anderen Fällen gestaltet sich die Isolation des Produktes schwierig.

### f. Anionenaustausch an einem lonentauscherharz

Der Austausch des Ions [X⁻] gegen das gewünschte Ion [A⁻] kann auch in einem allgemein bekannten Verfahren an einem herkömmlichen Anionentauscherharz erfolgen; aus ökonomischer Sicht ist dieses Verfahren im Industriemaßstab aber kaum anwendbar, da die maximale Beladung eines Harzes sehr beschränkt ist.

### g. Alkylierung bzw. Arylierung zu einem quaternären Carbonat

Hier wird das Amin R₃N mit dem Alkylierungs- oder Arylierungsmittel Dialkyl- oder Diarylcarbonat R'₂CO₃ umgesetzt, wobei ein quatemäres Alkyl- oder Arylcarbonat [NR'R₃⁺][R'CO₃⁻] gebildet wird. Dieses kann selbst bereits die gewünschte ionische Verbindung sein oder als Edukt zur Synthese der Verbindung [NR'R₃'][A⁻] dienen, wie in den nun folgenden Punkten h. bis j. beschrieben. Weiterhin sind aufgrund des Herstellungsprozesses die so erhaltenen Verbindungen frei von (korrosiven) Halogeniden.

### h. Anionenaustausch durch Reaktion mit einer Brønsted-Säure

Das gewünschte Anion [A⁻] wird bevorzugt durch Reaktion des quaternären Carbonates [NR'R₃⁺][R'CO₃⁻] mit der zum Anion [A⁻] konjugierten Brønsted-Säure HA eingeführt, wobei Kohlendioxid freigesetzt wird. Diese Reaktion ist äußerst vielseitig, schnell, einfach und effizient und erzeugt keinen festen oder flüssigen Abfall.

### i. Anionenaustausch via Metathese

Alternativ zu der bevorzugten Reaktion mit einer Brønsted-Säure HA, kann auch eine Metathese analog zu d. durchgeführt werden, wobei nun das quaternäre Carbonat [NR'R₃⁺][R'CO₃⁻] mit einem Metallsalz M⁺A⁻ umgesetzt wird, wobei das Kation des Metallsalzes M⁺ mit dem Carbonat [R'CO₃⁻] schwerlösliches [M⁺][R'CO₃⁻] bildet, welches ausfällt und entfernt wird, sodaß die gewünschte ionische Verbindung [NR'R₃⁺[A⁻] zurückbleibt und isoliert werden kann. Typische Metalle M⁺ sind z.B. Calcium, Magnesium, Zink, Mangan etc. Diese Reaktion verläuft nahezu quantitativ in wäßriger Lösung und benötigt im Gegensatz zu d. kein organisches Lösungsmittel.

### j. Anionenaustausch mittels Flüssigionenaustausch

Eine weitere Möglichkeit besteht in der Reaktion eines langkettigen Carbonats [NR'R₃⁺][R'CO₃⁻], welches im Allgemeinen wegen der hohen Polarität des Anions [R'CO₃⁻] wasserlöslich ist, mit einem Metallsalz M⁺A⁻, wobei hier das Kation M⁺ keine schwerlöslichen Carbonate bildet; es kommt zu einer Ionenaustauschreaktion und wasserunlösliches [NR'R₃⁺][A⁻] scheidet sich ab, während [M⁺][R'CO₃⁻] in Lösung bleibt. Typischerweise gelingt diese Reaktion mit einer Vielzahl von Anionen A⁻, welche in der Regel immer wesentlich unpolarer sind als das Anion [R'CO₃⁻], sodaß also das Ionenpaar [NR'R₃⁺][A⁻] nicht mehr wassermischbar ist und sich abscheidet. Die beschriebenen Verfahren zur Herstellung Ionischer Verbindungen, also ionischer Flüssigkeiten oder ionischer Feststoffe, weisen alle ihre typischen Nachteile auf, so dass weiterhin ein Bedarf an Verfahren besteht, die diese Nachteile nicht aufweisen.

Es wurde nun gefunden, dass 1,3-heteroaromatische 2-Carboxylate der Formel Ia oder Gemische von 1,3-heteroaromatischen 2-Carboxylaten der Formel Ia mit 1,3-heteroaromatischen Alkylcarbonaten und/oder 1,3-heteroaromatischen Arylcarbonaten der Formel IIa mit Wasser zu 1,3-heteroaromatischen Hydrogencarbonaten der Formel IIIa und/oder 1,3-heteroaromatischen Carbonaten der Formel IVa umgesetzt werden können, welche ggf. als Edukte für die Darstellung von ionische Verbindungen der Formel XXVIIa und/oder XXVIIb eingesetzt werden können: 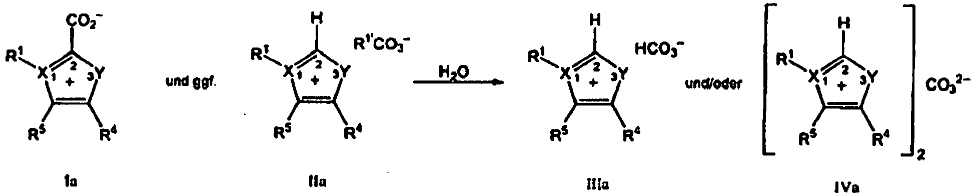
- wobei diese Hydrolyse in Abwesenheit von Brønsted-Säuren oder Brønsted-Basen durchgeführt wird. Abwesenheit bedeutet, daß bis zu 1mol% Brønsted-Säure bezogen auf die Summe der 1,3-heteroaromatischen 2-Carboxylate (Ia), und 1,3-heteroaromatischen Alkylcarbonate und 1,3-heteroaromatischen Arylcarbonate (IIa), anwesend sein können, und bis zu 1mol% Brønsted-Base zusätzlich zu den Anionen R^{1'}CO₃⁻, bezogen auf die Summe der 1,3-heteroaromatischen 2-Carboxylate (Ia), und 1,3-heteroaromatischen Alkylcarbonate und 1,3-heteroaromatischen Arylcarbonate (IIa), anwesend sein können,
- wobei X Stickstoff (N) ist und Y Sauerstoff (O), Schwefel (S), oder eine N-R³, P-R³, Gruppe ist.
- wobei die Reste R¹, R^{1'} und R³ voneinander unabhängig lineare, cyclische, verzweigte, gesättigte oder ungesättigte Alkylreste, mono- oder polycyclische aromatische oder heteroaromatische Reste dieser Reste sind, und R³ zusätzlich Wasserstoff sein kann,
- wobei die Reste R⁴ und R⁵ voneinander unabhängig Wasserstoff, Halogen, Cyano, Nitro, -SO₃H, -COOH, -OPO₂HR^{a}, -OPO₃R^{a}H, -OSO₃H, sowie die Salze der fünf letztgenannten Reste, -OSO₃R^{a}, -SO₃R^{a}, -CO₂R^{a}, -OPO₂R^{a}R^{b}, -O-R^{a}, -SR^{a}_{.} -NR^{a}R^{b}, -CONR^{a}R^{b}, -SO₂NR^{a}R^{b} oder gegebenenfalls substituierte lineare, cyclische, verzweigte, gesättigte oder ungesättigte Alkylreste, gegebenenfalls substituierte mono- oder polycyclische aromatische oder heteroaromatische Reste sind und R^{a} und R^{b} voneinander unabhängig für Wasserstoff, C₁-C₆-Alkyl, C₁-C₆-Halogenalkyl, Phenyl, Benzyl, Tolyl, Cyclopentyl oder Cyclohexyl stehen, oder
- wobei zwei benachbarte Reste der Reste R³,R⁴,R⁵ mit den Ringatomen, an welche sie gebunden sind, ein cyclisches oder aromatisches Ringsystem bilden, welche ggf. substituiert sein können, oder
- im Falle der 1,3-heteroaromatischen Alkylcarbonate oder 1,3-heteroaromatischen Arylcarbonate (IIa) R¹ und R^{1'} gemeinsam eine -CHR^{c}-CHR^{d}-Kette, - CHR^{c}-CH₂-CHR^{d}-Kette oder ggf. substuierten -C₆H₄-1,2-diyl-Rest bilden, wobei R^{c} und R^{d} unabhängig voneinander für H oder C₁-C₆-Alkyl stehen,
- wobei in den Verbindungen Ia, IIa, IIIa, IVa, voneinander unabhängig an Stelle eines Strukturelements, ausgewählt aus der Gruppe C-R⁴ und C-R⁵, N stehen kann,
   wobei
- R¹, R^{1'} voneinander unabhängig für C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl-, Heteroaryl-, C3-C7-Cycloalkyl, Halogen, OR^{a}, SR^{a}, NR^{a}R^{b}, COOR^{a}, CO-NR^{a}R^{b}, COR^{a}, oder OR^{a}, wobei R^{a} für Wasserstoff, C₁-C₆-Alkyl, C₁-C₆-Halogenalkyl, Phenyl, Benzyl, Tolyl, Cyclopentyl, Cyclohexyl, steht,
   vorzugsweise C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
   C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl; CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
   C₃- bis C₁₂-Cycloalkyl, insbesondere Cyclopentyl oder Cyclohexyl, C₃- bis C₁₂-Cycloalkyl, welches durch C₁-C₆₋Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
   CₙF_{2(n-a)-(1-b)}H_{2a-b} mit n ≤30, 0 ≤ a ≤ n und b = 0 oder 1;
   C₂- bis C₃₀-Alkenyl, insbesondere 2-Propenyl, 3-Butenyl, cis-2-Butenyl, trans-2-Butenyl, oder CₙF_{2(n-a)-(1-b)}H_{2a-b} mit n ≤30, 0 ≤ a ≤ n und b = 0 oder 1;
   C₃- bis C₁₂-Cycloalkenyl, insbesondere 3-Cyclopentenyl, 2-Cyclohexenyl, 3-Cyclohexenyl, 2,5-Cyclohexadienyl;
   CₙF_{2(n-a)-(1-b)}H_{2a-b} mit n ≤30, 0 ≤ a ≤ n und b = 0 oder 1,
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
   Aryl oder Heteroaryl mit 2 bis 30 Kohlenstoffatomen, welches durch ein bis drei C₁-C₆-Alkyl- oder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
   oder C₆F₍₅₋ₐ₎Hₐ mit 0≤ a ≤ 5;
   bedeuten,
- R³ Wasserstoff, sowie die unter R^{1'} genannten Reste, vorzugsweise Wasserstoff oder die unter R^{1'} vorzugsweise genannten Reste,
- R⁴, R⁵ für Wasserstoff, Halogen, OR^{a}, COR^{a}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl, Heteroaryl, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl, Cyclohexyl, steht;
   vorzugsweise für Wasserstoff;
   Halogen;
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl;
   C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
   CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
   OR^{a}, insbesondere Methoxy, Ethoxy, oder
   COR^{a}, insbesondere Formyl oder Acetyl,
   C₃- bis C₁₂₋Cycloalkyl, insbesondere Cyclopentyl oder Cyclohexyl,
   C₃- bis C₁₂-Cycloalkyl,welches durch C₁-C₆₋Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
   CₙF_{2(n-a)-(1-b)}H_{2a-b} mit n ≤30, 0 ≤ a ≤ n und b = 0 oder 1;
   C₂₋ bis C₃₀-Alkenyl, insbesondere 2-Propenyl, 3-Butenyl, cis-2-Butenyl, trans-2-Butenyl, oder CₙF_{2(n-a)-(1-b})H_{2a-b}mit n ≤30, 0 ≤ a ≤ n und b = 0 oder 1;
   C₃- bis C₁₂-Cycloalkenyl, insbesondere 3-Cyclopentenyl, 2-Cyclohexenyl, 3-Cyclohexenyl, 2,5-Cyclohexadienyl;
   CₙF_{2(n-a)-(1-b)}H_{2a-b}mit n ≤30, 0 ≤ a ≤ n und b = 0 oder 1,
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
   Aryl oder Heteroaryl mit 2 bis 30 Kohlenstoffatomen, welches durch ein bis drei C₁-C₆-Alkyl- opder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
   oder C₆F(₅₋ₐ)Hₐ mit 0≤ a ≤ 5;
   bedeuten; oder
- zwei benachbarte Reste der Reste R³,R⁴,R⁵ bilden mit den Ringatomen, an welchen sie gebunden sind, ein 5- gliedriges Ringsystem oder ein 5-gliedriges aromatisches Ringsystem, welche unsubstituiert sind, oder substituiert sind durch 1 bis 4 Reste aus der Gruppe C₁-C₆-Alkyl, Aryl, Heteroaryl, C₃-C₇-Cycloalkyl, Halogen, OR^{a}, SR^{a}, NR^{a}R^{b}, COOR^{a}, CO-NR^{a}R^{b} oder COR^{a},wobei R^{a} und R^{b} unabhängig voneinander für Wasserstoff, C₁-C₆-Alkyl, C₁-C₆-Halogenalkyl, Phenyl, Benzyl, Tolyl, Cyclopentyl, Cyclohexyl, steht, oder
- im Falle der 1,3-heteroaromatischen Alkylcarbonate oder 1,3-heteroaromatischen Arylcarbonate (IIa) kann R¹ und R¹' gemeinsam eine -CHR^{c}-CHR^{d}-Kette, -CHR^{c}-CH₂-CHR^{d}-Kette oder ggf. substuierten -C₆H₄-1,2-diyl-Rest bilden, wobei R^{c} und R^{d} unabhängig voneinander für H oder C₁-C₆-Alkyl stehen.

In einer weiteren Ausgestaltungsform werden 1,3-heteroaromatische 2-Carboxylate der Formel la oder Gemische von 1,3-heteroaromatischen 2-Carboxylaten der Formel la mit 1,3-heteroaromatischen Alkylcarbonaten und/oder 1,3-heteroaromatischen Arylcarbonaten der Formel IIa eingesetzt, wobei
- R¹ R¹' voneinander unabhängig für C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b}, COOR^{a}, oder OR^{a}, wobei R^{a} und R^{b} voneinander unabhängig für Wasserstoff, C₁-C₆Alkyl, C₁-C₆-Halogenalkyl, Phenyl, Benzyl, Tolyl, Cyclopentyl und Cyclohexyl steht,
   vorzugsweise C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
   C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
   CₙF_{2(n-a)(1-b)}H_{2a-b}mit n ≤30, 0 ≤ a ≤ n und b = 0 oder 1, insbesondere CF₃, C₂F₅, C₆F₁₃,
   C₈F₁₇, C₁₀F₂₁, C₁₂F₂₅; oder
   C₃- bis C₁₂-Cycloalkyl, insbesondere Cyclopentyl oder Cyclohexyl,
   C₃- bis C₁₂-Cycloalkyl,welches durch C₁-C₆Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
   CnF_{2(n-a)-(1-b})H_{2a-b}mit n ≤30, 0 ≤ a ≤ n und b = 0 oder 1;
   C₂- bis C₃₀-Alkenyl, insbesondere 2-Propenyl, 3-Butenyl, cis-2-Butenyl, trans-2-Butenyl, oder CₙF_{2(n-a)-(1-b})H_{2a-b} mit n ≤30, 0 ≤ a ≤ n und b = 0 oder 1;
   C₃- bis C₁₂-Cycloalkenyl, insbesondere 3-Cyclopentenyl, 2-Cyclohexenyl, 3-Cyclohexenyl, 2,5-Cyclohexadienyl;
   CₙF_{2(n-a)-(1-b)}H_{2a-b} mit n ≤30, 0 ≤ a ≤ n und b = 0 oder 1,
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
   Aryl oder Heteroaryl mit 2 bis 30 Kohlenstoffatomen, welches durch ein bis drei C₁-C₆-Alkyl- oder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
   oder C₆F₍₅₋ₐ₎Hₐ mit 0≤ a ≤ 5;
   bedeuten.
- R³ Wasserstoff, sowie die unter R^{1'} genannten Reste, vorzugsweise Wasserstoff oder die unter R^{1'} vorzugsweise genannten Reste,
- R⁴, R⁵ Wasserstoff, Halogen, OR^{a}, COR^{a}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl, Heteroaryl, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} für Wasserstoff, C₁-C₆-Alkyl, C₁-C₆-Halogenalkyl, Phenyl, Benzyl, Tolyl, Cyclopentyl und Cyclohexyl steht,
   vorzugsweise für Wasserstoff;
   Halogen;
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl;
- C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
   CₙF_{2(n-a)-(1-b)}H_{2a'b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
   OR^{a}, insbesondere Methoxy, Ethoxy, oder
   COR⁸, insbesondere Formyl oder Acetyl,
   C₃- bis C₁₂-Cycloalkyl, insbesondere Cyclopentyl oder Cyclohexyl,
   C₃- bis C₁₂-Cycloalkyl,welches durch C₁-C₆₋Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
   CₙF_{2(n-a)o(1-b)}H_{2a-b} mit n ≤30, 0 ≤ a ≤ n und b = 0 oder 1;
   C₂- bis C₃₀-Alkenyl, insbesondere 2-Propenyl, 3-Butenyl, cis-2-Butenyl, trans-2-Butenyl, oder CₙF_{2(n-a)-(1-b)}H_{2a-b} mit n ≤30, 0 ≤ a ≤ n und b = 0 oder 1;
   C₃- bis C₁₂-Cycloalkenyl, insbesondere 3-Cyclopentenyl, 2-Cyclohexenyl, 3-Cyclohexenyl, 2,5-Cyclohexadienyl;
   CₙF_{2(n-a)-(1-b)}H_{2a-b}mit n ≤30, 0 ≤ a ≤ n und b = 0 oder 1,
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
   Aryl oder Heteroaryl mit 2 bis 30 Kohlenstoffatomen, welches durch ein bis drei C₁-C₆-Alkyl- opder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
   oder C₆F₍₅₋ₐ₎Hₐ mit 0≤ a ≤ 5;
   bedeuten; oder
- zwei benachbarte Reste der Reste R³,R⁴,R⁵ bilden mit den Ringatomen, an welchen sie gebunden sind, ein 5- gliedriges Ringsystem oder ein 5-gliedriges aromatisches Ringsystem, welche unsubstituiert sind, oder substituiert sind durch 1 bis 4 Reste aus der Gruppe C₁-C₆-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl und Cyclohexyl, stehen.

In einer weiteren Ausgestaltungsform werden 1,3-heteroaromatische 2-Carboxylate der Formel la oder Gemische von 1,3-heteroaromatischen 2-Carboxylaten der Formel la mit 1,3-heteroaromatischen Alkylcarbonaten und/oder 1,3-heteroaromatischen Arylcarbonaten der Formel IIa eingesetzt,
- wobei im Falle der 1,3-heteroaromatischen Alkylcarbonate oder 1,3-heteroaromatischen Arylcarbonate (IIa) R¹ und R^{1'} gemeinsam eine -CHR^{c}-CHR^{d}-Kette,
- -CHR^{c}-CH₂-CHR^{d}-Kette oder ggf. substuierten -C₆H₄-1,2-diyl-Rest bilden, wobei R^{c} und R^{d} unabhängig voneinander für H oder C₁-C₆-Alkyl stehen.

In einer weiteren Ausgestaltungsform werden 1,3-heteroaromatische Carboxylate der Formel Ia eingesetzt, wobei das 1,3-heteroaromatische 2-Carboxylat bevorzugt aus der Gruppe der folgenden Strukturen ausgewählt ist:

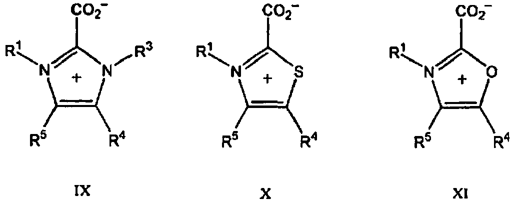

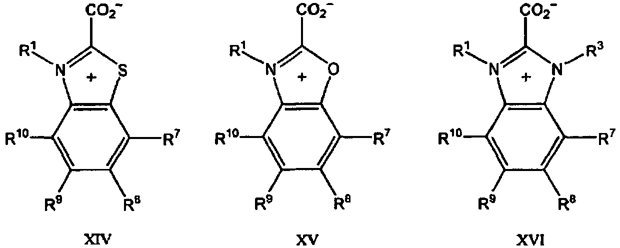

Wobei die Reste R⁷ bis R¹⁰ folgende Bedeutung haben: Wasserstoff, C₁-C₆-Alkyl, Aryl, Heteroaryl, C₃-C₇-Cycloalkyl, Halogen, -OR^{a}, -SR^{a}, -NR^{a}R^{b}, -COOR^{a}, CO-NR^{a}R^{b} oder -COR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl und Cyclohexyl stehen, und die Reste R¹, R⁴ bis R⁶ die voranstehenden Bedeutungen haben.

1,3-heteroaromatische 2-Carboxylate der Formel la oder Gemische von 1,3-heteroaromatischen 2-Carboxylaten der Formel la mit 1,3-heteroaromatischen Alkylcarbonaten und/oder 1,3-heteroaromatischen Arylcarbonaten der Formel IIa werden mit Wasser umgesetzt,
- wobei das Wasser äquimolar oder im Überschuß, bezogen auf die Summe des 1,3-heteroaromatischen 2-Carboxylats la und ggf. 1,3-heteroaromatischem Alkylcarbonats und/oder 1,3-heteroaromatischem Arylcarbonats IIa, zugegeben werden kann. Üblicherweise wird eine Menge von 0,5 bis 20 Molequivalenten
   Wasser, bezogen auf die Summe des 1,3-heteroaromatischen 2-Carboxylats und ggf. 1,3-heteroaromatischem Alkylcarbonats und/oder 1,3-heteroaromatischem Arylcarbonats, bevorzugt 2 bis 5 Molequivalenten Wasser, besonders bevorzugt 3 Molequivalenten Wasser, zugegeben.

Üblichwerweise werden die 1,3-heteroaromatischen 2-Carboxylate der Formel Ia oder Gemische von 1,3-heteroaromatischen 2-Carboxylaten der Formel la mit 1,3-heteroaromatischen Alkylcarbonaten und/oder 1,3-heteroaromatischen Arylcarbonaten der Formel IIa mit Wasser bei einer Temperatur von 30°C bis 350°C, bevorzugt 50°C bis 150°C, besonders bevorzugt 80°C bis 100°C, umgesetzt.

In der Regel werden 1,3-heteroaromatische 2-Carboxylate der Formel Ia oder Gemische von 1,3-heteroaromatischen 2-Carboxylaten der Formel Ia mit 1,3-heteroaromatischen Alkylcarbonaten und/oder 1,3-heteroaromatischen Arylcarbonaten der Formel IIa mit Wasser für eine Zeitdauer von 1 Minute bis 7 Tagen, bevorzugt 3 Stunden bis 2 Tagen, besonders bevorzugt 5 Stunden bis 12 Stunden, umgesetzt.

Die Umsetzung erfolgt bei einem Druck von 1 bis 20 bar, vorzugsweise bei dem Eigendruck des Reaktionsgemisches.

Die Umsetzung kann in Substanz durchgeführt werden oder in Gegenwart eines Lösungsmittels wie eines Alkohols, insbesondere wie Methanol, Ethanol, Isopropanol, n-Propanol, n-Butanol, sec-Butanol, tert-Butanol, Pentanol, Hexanol, Heptanol oder Octanol, eines Ethers, wie Diethylether oder Tetrahydrofuran, eines Dialkylformamids, wie Dimetylformamid, Diethylformamid, eines Ketons, wie Aceton, Methylethylketon, eines Sulfoxids, wie Dimethylsulfoxid, oder eines Nitrils, wie Acetonitril, oder eines Gemisches hiervon.

Die Umsetzung kann batchweise, semikontinuierlich oder kontinuierlich erfolgen.

Üblicherweise wird die Umsetzung unter Durchmischung der eingesetzten Reagentien durchgeführt. Dies erfolgt nach dem Fachmann bekannten Methoden, wie z.B. Rühren oder Schütteln.

Die Aufarbeitung erfolgt üblicherweise dadurch, daß das Wasser teilweise und/oder das Lösungsmittel gänzlich oder teilweise durch Destillation, Vakuumdestillation, Dünnschichtverdampfung, Kurzwegverdampfung, Rotations-verdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren entfernt wird.

Es ist möglich, die Umsetzung bzw. Aufarbeitung unter Luft- oder Wasserdampfatmosphäre oder unter Schutzgasatmosphäre, beispielsweise unter Stickstoff, Kohlendioxid oder Argon. durchzuführen.

In einer weiteren Ausgestaltungsform der vorliegenden Erfindung wird die Umsetzung des 1,3-heteroaromatischen Carboxylats der Formel Ia bzw. des Gemisches von 1,3-heteroaromatischem Carboxylat der Formel Ia und 1,3-heteroaromatischem Alkyl- und/oder Arylcarbonat der Formel IIa in Gegenwart eines Katalysators durchgeführt, wobei dieser Katalysator keine Brønstedsäure oder - Base ist. Dieser Katalysator enthält in der Regel mindestens ein Element der 3. bis 13. Gruppe des Periodensystems und/oder der Gruppe der Lanthanoiden (Atomnummer 57 bis 71), wobei dieser Katalysator keine Brønstedsäure oder - Base ist.

Dieser Katalysator kann im Reaktionsgemisch homogen gelöst, suspendiert, heterogen als Feststoff oder auf einem Trägermaterial imobilisert vorliegen.

Vorzugsweise enthält der Katalysator mindestens ein Element ausgewählt aus der Gruppe Ruthenium, Osmium, Cobalt, Rhodium, Iridium. Nickel, Palladium, Platin, Titan, Zirkon, Vanadium, Mangan, Scandium, Chrom, Molybdän, Wolfram, Eisen, Kupfer, Silber, Aluminium, Lanthan, Cer, Neodym, Samarium, Gadolinium, Erbium und Lutetium.

Üblicherweise werden von 0,000001 bis 90mol%, vorzugsweise 0,001 bis 10mol%, besonders bevorzugt 0,1 bis 1 mol% Katalysator bezogen auf das 1,3-heteroaromatische Carboxylat der Formel Ia bzw. der Summe von 1,3-heteroaromatischem Carboxylat der Formel Ia und 1,3-heteroaromatischem Alkyl- oder Arylcarbonat der Formel IIa eingesetzt.

Die Katalysatoren sind handelsüblich bzw. können aus handelsüblichen Chemikalien hergestellt werden.

Die Verbindungen der Formel Ia und **II**a werden durch Umsetzung eines 1,3-Heteroaromaten der Formel XVII und/oder XVIII, wobei die Reste X,Y,Z, und R⁴ bis R⁶, die Bedeutung wie für die Verbindungen der Formel Ia haben, mit einem Alkyl-oder Arylcarbonat R¹OCOOR^{1'}, wobei die Reste R¹ und R^{1'}die Bedeutung wie bereits weiter oben beschrieben, erhalten.

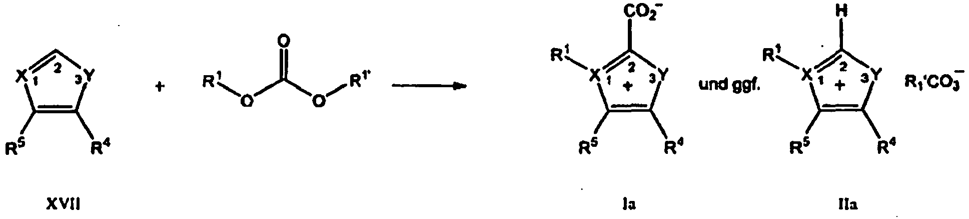

Bevorzugt werden hierbei 1,3-Heteroaromaten der folgenden Strukturen eingesetzt, wobei die Bedeutungen der Resten R³ bis R¹⁰ wie voranstehend definiert ist:

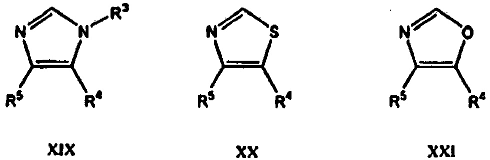

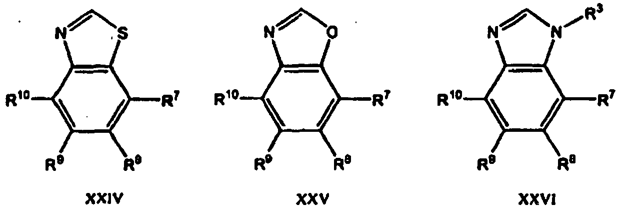

Der 1,3-Heteroaromat (XVII) und das Dialkylcarbonat und/oder Diarylcarbonat und/oder Alkylarylcarbonat R¹OCOOR^{1'} werden in einem Molverhältnis von 0,1 bis 20 Molequivalenten R¹OCOOR^{1'} bezogen auf die Verbindung XVII, bevorzugt 0,5 bis 5 Molequivalenten, besonders bevorzugt 0,8 bis 2 Molequivalenten, ganz besonders bevorzugt 0.95 bis 1,2 Molequivalenten, umgesetzt.

Die Umsetzung erfolgt bei einer Temperatur von 30°C bis 350°C, bevorzugt 50°C bis 200°C, besonders bevorzugt 80°C bis 160°C, und ganz besonders bevorzugt 60°C bis 130°C**.**

Die Umsetzung nimmt eine Zeitdauer von 1 Minute bis 14 Tagen, bevorzugt 3 Stunden bis 5 Tagen, besonders bevorzugt 6 Stunden bis 24 Stunden in Anspruch.

Die Umsetzung erfolgt bei einem Druck von 1 bis 50 bar, vorzugsweise bei dem Eigendruck des Reaktionsgemisches.

Die Umsetzung kann in Substanz durchgeführt werden oder in Gegenwart eines Lösungsmittels wie eines Alkohol, insbesondere wie Methanol, Ethanol, Isopropanol, n-Propanol, n-Butanol, sec-Butanol, tert-Butanol, Pentanol, Hexanol, Heptanol oder Octanol, eines Ethers, wie Diethylether oder Tetrahydrofuran, eines Dialkylformamids, wie Dimetylformamid, Diethylformamid, eines Ketons, wie Aceton, Methylethylketon, eines Sulfoxids, wie Dimethylsulfoxid, oder eines Nitrils, wie Acetonitril, oder eines Gemisches hiervon.

Die Umsetzung kann batchweise, semikontinuierlich oder kontinuierlich erfolgen.

Üblicherweise wird die Umsetzung unter Durchmischung der eingesetzten Reagentien durchgeführt. Dies erfolgt nach dem Fachmann bekannten Methoden, wie z.B. Rühren oder Schütteln.

Die Aufarbeitung erfolgt üblicherweise dadurch, daß das Lösungsmittel und/oder das nicht umgesetzte Edukt XVII und/oder das nicht umgesetzte Edukt R¹OCOOR^{1'} gänzlich oder teilweise durch Destillation, Vakuumdestillation, Dünnschichtverdampfung, Kurzwegverdampfung, Rotations-Verdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren entfernt wird.

Das nicht umgesetzte Edukt XVII kann auch durch Extraktion mit einem Lösungsmittel, z.B. einem Kohlenwasserstoff wie Pentan, Hexan, Heptan, Octan, Nonan, Decan, Petrolether, Benzin, Diesel, Benzol, Toluol, o-Xylol, m-Xylol, p-Xylol, Ether wie Diethylether, Tetrahydrofuran, Ester wie Ethylacetat, Methylacetat, chlorierte Kohlenwasserstoffe wie Chloroform, Dichlormethan, oder eines Gemisches derselben, entfernt werden, wobei zusätzlich ein Hilfslösungsmittel wie ein Alkohol, insbesondere wie Methanol, Ethanol, Isopropanol, n-Propanol, n-Butanol, sec-Butanol, tert-Butanol, Pentanol, Hexanol, Heptanol oder Octanol, ein Ether, wie Diethylether oder Tetrahydrofuran, ein Dialkylformamid, wie Dimetylformamid, Diethylformamid, ein Keton, wie Aceton, Methylethylketon, ein Sulfoxid, wie Dimethylsulfoxid, oder ein Nitril, wie Acetonitril, oder ein Gemisches hiervon, zugegeben werden kann.

Es ist möglich, die Umsetzung bzw. Aufarbeitung unter Luft- oder Wasserdampfatmosphäre oder unter Schutzgasatmosphäre, beispielsweise mit Stickstoff, Kohlendioxid oder Argon, durchzuführen.

In einer Ausgestaltungsform der vorliegenden Erfindung wird das Reaktionsgemisch aufgearbeitet und anschließend das so erhaltene 1,3-heteroaromatische 2-Carboxylat der Formel Ia oder das Gemisch von 1,3-heteroaromatischen 2-Carboxylat mit 1,3-heteroaromatischen Alkylcarbonat und/oder 1,3-heteroaromatischen Arylcarbonat der Formel IIa mit Wasser umgesetzt.

In einer weiteren Ausgestaltungsform der vorliegenden Erfindung wird das Reaktionsgemisch nicht aufgearbeitet, oder nur ein Teil des bei der Reaktion gebildeten Alkohols entfernt oder falls in einem Lösungsmittel gearbeitet, nur ein Teil des Lösungsmittels entfernt, und dieses Reaktionsgemisch dann direkt mit Wasser behandelt.

Weiterhin wurde gefunden, daß Gemische von 1,3-heteroaromatischem Hydrogencarbonat der Formel IIIa und/oder 1,3-heteroaromatischem Carbonat der Formel IVa, mit Wasser lagerstabil sind. Diese Gemische enthalten in der Regel 0,5 bis 10, vorzugsweise 0,9 bis 2,0 Molequivalente Wasser. Die Herstellung der Gemische erfolgt durch Zugabe der gewünschten Menge an Wasser zu den isolierten Verbindungen der Formel IIIa und/oder IVa oder indem bei der Aufarbeitung des Reaktionsansatzes die Entfernung des Wassers unterbrochen wird, wenn der gewünschte Wassergehalt erreicht ist.

Ferner betrifft die vorliegende Erfindung ein Verfahren zur Herstellung von Verbindungen der Formel XXVIIa, wobei Aⁿ⁻ (n > 0) für alle Anionen steht, welche in Form ihrer stabilen oder metastabilen konjugierten Brørnsted-Säuren HₙA, der entsprechenden Säureanhydride der Brønsted-Säuren HₙA sowie aller Salze [Kation^{m+}]ₙ[Aⁿ⁻]ₘ (m > 0) deren Löslichkeitsprodukt größer ist als das Löslichkeitsprodukt [Kation^{m+}][HCO₃⁻]ₘ und/oder [Kation^{m+}]₂[CO₃²⁻]ₘ,
wobei in einem Schritt A das 1,3-heteroaromatische Carboxylat der Formel Ia oder ein Gemisch von 1,3-heteroaromatischem Carboxylat der Formel Ia und 1,3-heteroaromatischem Alkyl- oder Arylcarbonats der Formel IIa mit Wasser umgesetzt wird 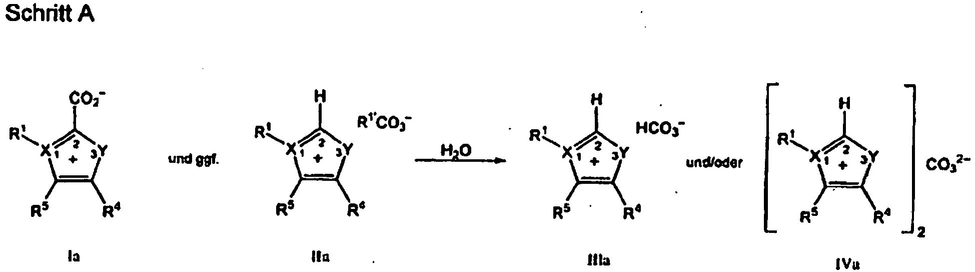 und das so hergestellte 1,3-heteroaromatische Hydrogencarbonat der Formel IIIa und/oder 1,3-heteroaromatische Carbonat der Formel IVa wird dann im Schritt B zu der Verbindung der Formel XXVIIa umgesetzt.

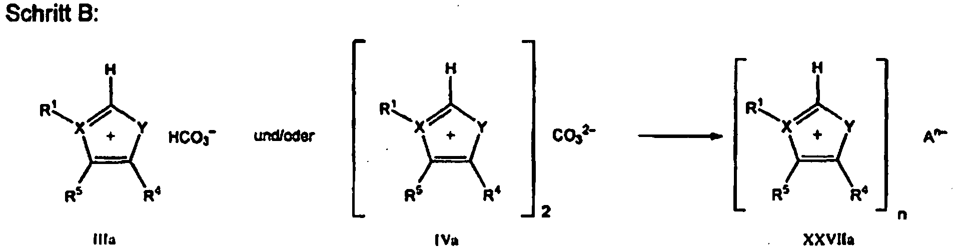

Die Herstellung der Verbindungen der Formel XXVIIa aus 1,3-heteroaromatischem Hydrogencarbonat der Formel IIIa und/oder 1,3-heteroaromatischem Carbonat der Formel IVa kann dabei wie folgt durchgeführt werden:

### Anionenaustausch durch Reaktion mit einer Brønsted-Säure:

Das gewünschte Anion [Aⁿ⁻] wird bevorzugt durch Reaktion des 1,3-heteroaromatischen Hydrogencarbonats der Formel IIIa und/oder 1,3-heteroaromatischen Carbonats der Formel IVa mit der zum Anion [Aⁿ⁻] konjugierten Brønsted-Säure HₙA eingeführt, wobei Kohlendioxid freigesetzt wird. Diese Reaktion ist äußerst vielseitig, schnell, einfach und effizient und erzeugt keinen festen oder flüssigen Abfall.

### Anionenaustausch via Metathese

Alternativ zu der bevorzugten Reaktion mit einer Brønsted-Säure HₙA, kann auch eine Metathese durchgeführt werden, wobei das 1,3-heteroaromatische Hydrogencarbonat der Formel IIa und/oder 1,3-heteroaromatische Carbonat der Formel IVa mit einem Metallsalz [Kation^{m+}]ₙ[Aⁿ⁻]ₘ = [M^{m+}]ₙ[Aⁿ⁻]ₘ umgesetzt wird, wobei das Kation des Metallsalzes M^{m+} mit dem Hydrogencarbonat und/oder Carbonat schwerlösliches M^{m+}-Hydrogencarbonat und/oder M^{m+}-Carbonat bildet, welches ausfällt und durch Filtration oder Zentrifugation entfernt wird, sodaß die gewünschte ionische Verbindung der Formel XXVIIa zurückbleibt und isoliert werden kann. Typische Metalle M^{m+} sind z.B. Calcium, Magnesium, Zink, Mangan etc. Diese Reaktion verläuft nahezu quantitativ in wäßriger Lösung.

### Anionenaustausch mittels Flüssigionenaustausch

Eine weitere Möglichkeit besteht in der Reaktion eines langkettigen 1,3-heteroaromatischen Hydrogencarbonats der Formel IIIa und/oder 1,3-heteroaromatischen Carbonats der Formel IVa mit einer C₄ bis C₃₀, bevorzugt C₆ bis C₁₆, besonders bevorzugt C₈ bis C₁₂ Alkylkette, welche ggf. verzweigt, funktionalisiert oder substituiert sein kann, mit einem Salz [Kation^{m+}]ₙ[Aⁿ⁻]ₘ, wobei hier das Kation^{m+} keine schwerlöslichen Hydrogencarbonate und/oder Carbonate bildet; es kommt zu einer Ionenaustauschreaktion und wasserunlösliches XXVIIa scheidet sich ab, während M^{m+}-Hydrogencarbonat und/oder M^{m+}-Carbonat ganz oder teilweise in Lösung bleibt. M^{m+} ist üblicherweise ein Metall-Kation, welches keine schwerlöslichen Hydrogencarbonate oder Carbonate bildet, wie Natrium oder Kalium, oder ein organisches Kation, wie N⁺R^{e}R^{f}R^{g}R^{h}, P⁺R^{e}R^{f}R^{g}R^{h}, S⁺R^{e}R^{f}R^{g}, wobei R^{e},R^{f},R^{g} und R^{h} unabhängig voneinander für Wasserstoff, C₁-C₆-Alkyl, C₁-C₆-Halogenalkyl, Phenyl, Benzyl, Tolyl, Cyclopentyl, Cyclohexyl steht. Typischerweise gelingt diese Reaktion mit einer Vielzahl von Anionen Aⁿ⁻, welche in der Regel immer wesentlich unpolarer sind als das Hydrogencarbonat oder Carbonat Anion.

Weiterhin betrifft die vorliegende Erfindung ein Verfahren zur Herstellung von Hydroxy-1-alkyl- oder Hydroxyaryl substituierten 1,3-heteroaromatischen Carboxylaten der Formel Ia, sowie ein Verfahren zur Herstellung von Hydroxy-1-alkyl- oder Hydroxyaryl substituierten 1,3-heteroaromatischen Hydrogencarbonaten der Formel IIIa und/oder Hydroxy-1-alkyl- oder Hydroxyaryl- substituierten 1,3-heteroaromatischen Carbonaten der Formel IVa dadurch gekennzeichnet, dass man einen 1,3-Heteroaromaten der Formel XVII mit einem cyclischen Carbonat der Formel R¹OCOOR^{1'} umsetzt 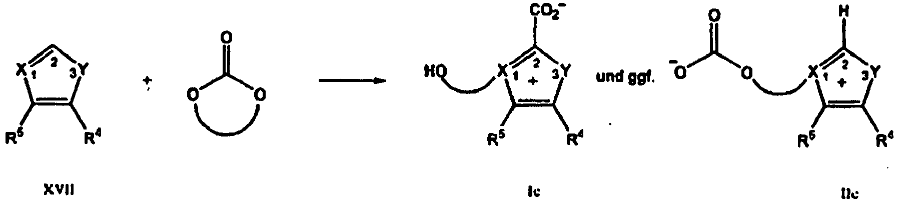
- wobei 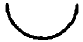 für -CHR^{c}-CHR^{d}-, -CHR^{c}-CH₂-CHR^{d}- oder ggf. substuierten -C₆H₄-1,2-diyl-Rest steht, wobei R^{c} und R^{d} unabhängig voneinander für H oder C₁-C₆-Alkyl stehen und die Reste X,Y,Z und R⁴ bis R⁶ die voranstehenden Bedeutungen haben,
   und ggf. anschließend das Hydroxy-1-alkyl- oder Hydroxyaryl substituierte 1,3-hetero-aromatische Carboxylat der Formel Ic und/oder Id und das ggf. gebildete Hydroxy-1-alkyl-oder Hydroxyaryl substituierte 1,3-heteroaromatische Alkyl- oder Arylcarbonat der Formel IIc und/oder IId mit Wasser hydrolysiert wird.

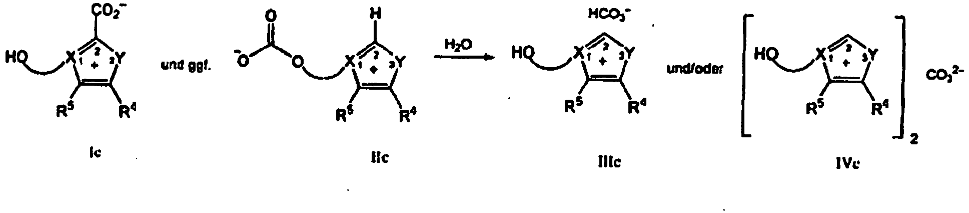

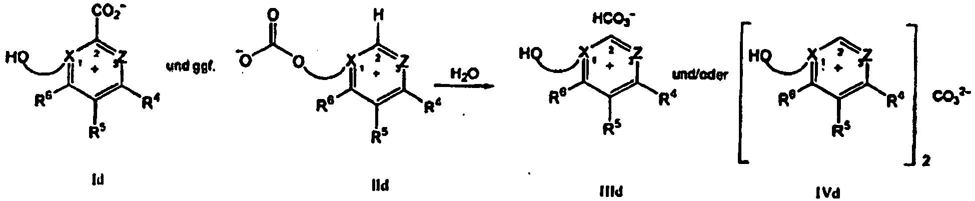

Die Bedingungen für die Umsetzungen gelten analog zu den voranstehenden Ausführungen. Vorzugsweise werden als cyclisch Carbonate Ethylencarbonat CAS 96-49-1. 1,2-Propylencarbonat CAS 108-32-7 oder 1,3-Propylencarbonat CAS 2453-03-4 eingesetzt. Weiterhin sind die entsprechenden 1,3-heteroaromatischen 2-Carboxylate Ic und/oder Id, 1,3-heteroaromatischen Carbonate IVc und/oder IVd und 1,3-heteroaromatischen Hydrogencarbonate IIIc und/oder IIId neu.

Die Erfindung betrifft ferner sämtliche 1,3-heteroaromatische 2-Carboxylate Ia, 1,3-heteroaromatische Hydrogencarbonate IIIa, 1,3-heteroaromatische Carbonate IVa oder Gemische derselben, die nach dem erfindungsgemäßen Verfahren hergestellt sind.

### Beispiele:

Im Folgenden wird die Erfindung durch einige typische Beispiele näher beschrieben. ¹H-Kernmagnetresonanzspektren (¹H-NMR) wurden auf einem Bruker Avance DPX 400 in d⁶-DMSO und D₂O gemessen, Gehaltsbestimmungen mit NMR werden mit G(NMR) abgekürzt. Elektrospray-Ionisations-Massenspektren (ESI-MS) wurden auf einem Bruker Esquire 3000 in wäßrig-methanolischer Lösung im positiv und negativ Mode gemessen, acidimetrische Gehaltsbestimmungen, abgekürzt mit G(T), wurden mit einem Metrohm Basic Titrino 794 mit gestellter 1N Salzsäure durchgeführt. Die relative molare Masse wird mit "Mᵣ" abgekürzt und in g/mol angegeben, das Molequivalent wird mit "equiv." abgekürzt. Sämtliche Ausgangschemikalien wurden bei Sigma-Aldrich bezogen.

### a. Hydrolyse von 1,3-Dimethylimidazolium-2-carboxylat zu 1,3-Dimethylimidazolium-hydrogencarbonat:

Es soll im Folgenden am Beispiel des 1,3-Dimethylimidazolium-2-carboxylats gezeigt werden, daß die Hydrolyse reiner 1,3-heteroaromatischer 2-Carboxylate ohne Zugabe von sauren oder basischen Katalysatoren oder sonstiger Hilfsstoffe zum gewünschten 1,3-heteroaromatischen Hydrogencarbonat - in diesem Falle das 1,3-Dimethylimidazolium-hydrogencarbonat-führt:

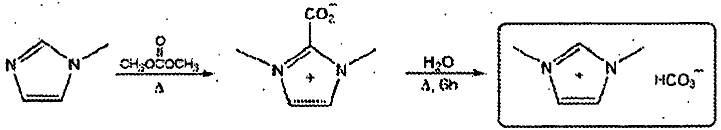

Darstellung des 1,3-Dimethylimidazolium-2-carboxylats: 41 g 1-Methylimidazol (CAS 616-47-7; 0,5mol) wurden mit 90g Dimethylcarbonat (CAS 616-38-6; 2,0 equiv.) und 50g Methanol versetzt und für 5 Tage refluxiert. Das Lösungsmittel und das überschüssige Dimethylcarbonat wurden *in vacuo* entfernt, der Rückstand am Hochvakuum getrocknet und 68,6g (98% der Theorie) einer weißen, kristallinen Substanz isoliert, welche mit 1N-Salzsäure nicht unter CO₂-Abgabe reagiert, nicht acidimetrisch titrierbar ist und damit kein freies 1,3-Dimethylimidazolium-Hydrogencarbonat, Methylcarbonat oder Carbonat enthält.

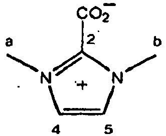

Summenformel: C₆H₈N₂O₂; Mᵣ= 140,1g/mol; ¹H-NMR (δ-Werte, 400MHz, D₂O): 3,903 (6H, s: Hₐ, H_{b}); 7,286 (2H, s: H₄, H₅); ESI-MS (+ mode, m/z) : 140,1 (M*); 163.0 (M*Na⁺) ; G(T): nicht titrierbar, da Substanz nicht pH-aktiv; keine Carbonate nachweisbar, da kein Verbrauch.

Hydrolyse des 1,3-Dimethylimidazolium-2-carboxylats: 40g (0,286mol) des 1,3-Dimethylimidazolium-2-carboxylats wurden in 20ml Wasser (4,0 equiv.) gelöst und für 6 Stunden auf 80°C erhitzt. Die abgekühlte Lösung enthielt ausschließlich die Substanz 1,3-Dimethylimidazolium-Hydrogencarbonat:

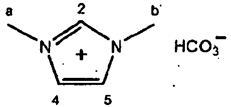

Summenformel: C₆H₁₀N₂O₃; Mᵣ = 158,2g/mol; ¹H-NMR (δ -Werte, 400MHz, D₂O): 3,792 (6H, s: Hₐ, H_{b}); 7,319 (2H, d, J_{4.2} und J_{5.2} nicht beobachtbar, da H₂ mit D₂O austauscht: H₄, H₅); 8,550 (Restsignal des nicht mit D ausgetauschten H₂); HC_{O3}⁻: nicht beobachtbar; ESI-MS (+ mode, m/z): 97,1(K⁺); (- mode, m/z): 61,0 (A⁻); G(T): 75,03% in Lösung nach der Hydrolyse, das entspricht 99,5% berechnet als isolierter Feststoff.

Da die Ausgangssubstanz 1,3-Dimethylimidazolium-2-carboxylat nicht titrierbar ist (und daher in einer acidimetrischen Titration nicht gemessen wird) und der als isolierter Feststoff berechnete Gehalt an 1,3-Dimethylimidazolium-Hydrogencarbonat bei 99,5% liegt, wurde somit das 1,3-Dimethylimidazolium-2-carboxylat nahezu quantitativ umgesetzt.

### b. Darstellung von 1-Butyl-3-methylimidazolium-hydrogencarbonat (BMIM-HCO₃):

Am Beispiel der Darstellung von BMIM-HCO₃ aus 1-Butylimidazol soll hier eine typische Synthese eines 1,3-heteroaromatischen Hydrogencarbonats beschrieben werden:

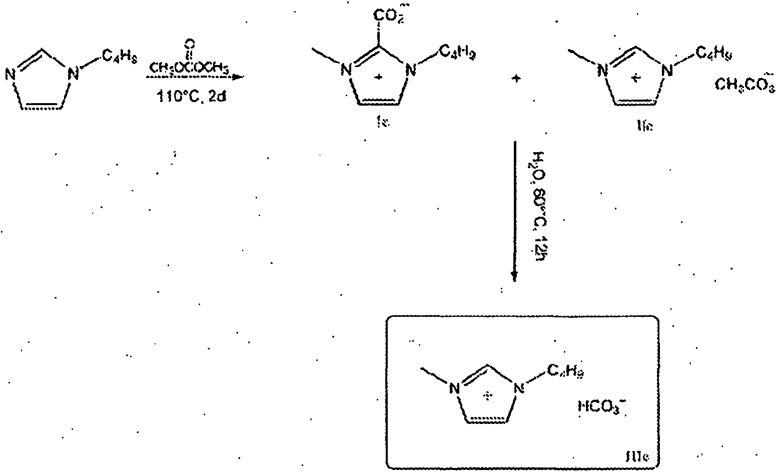

Stufe 1 - Methylierung des 1-Butylimidazols: 1200g 1-Butylimidazol (CAS 4316-42-1, 9,66mol), 1050g Dimethylcarbonat (CAS 616-38-6; 1,2 equiv.) und 1125g Methanol (33% der Reaktionsmischung) wurden in einem Druckreaktor mit Argon inertisiert und für 2 Tage auf 110°C unter Rühren erhitzt; dabei stellt sich typischerweise ein Systemeigenüberdruck von ca. 10 bar ein. Eine Probe wurde gezogen und *in vacuo* von Methanol und überschüssigem Dimethylcarbonat befreit. Die Charakterisierung dieser Probe ergab zwei Inhaltsstoffe - 1-Butyl-3-methylimidazolium-2-carboxylat (Ie) und 1-Butyl-3-methylimidazolium-methylcarbonät (Ile) - im Massenverhälnis von ca. 2 : 1 sowie einen praktisch quantitativen Reaktionsumsatz:

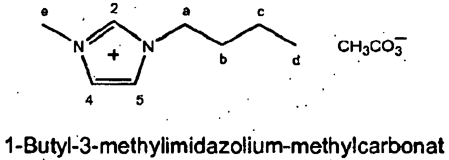

Summenformel: C₁₀H₁₈N₂O₂; Mᵣ = 214,1g/mol; ¹H-NMR (δ-Werte, 400MHz, d⁶-DMSO): 0,877 (3H, t, J_{d,c}=7,58Hz: H_{d}); 1,245 (2H, tq, J_{c,b}=7,54Hz, J_{c,d}=7,58Hz: H_{c}); 1,765 (2H, tt, J_{b,c}=7,54Hz, J_{b,a}=7,33Hz: H_{b}); 3,168 (3H, s: CH₃CO₃⁻); 3,859 (3H, s: Hₑ); 4,171 (2H, t, J_{a,b}=7,33Hz: Hₐ); 7,727 (1H, t, J_{4.5}=J_{4.2}=1,77Hz, B-Teil eines ABX-Systems: H₄*); 7,795 (1H, t, J_{5,4}=J_{5,2}=1,77Hz, A-Teil eines ABX-Systems: H₅*); 9,292 (1H, t, J_{2,4}=J_{2,5}=1,77Hz. X-Teil eines ABX-Systems: H₂); *Zuordnung vertauschbar; ESI-MS (+ mode, m/z): 139,1 (K⁺); (- mode, m/z): 75,0 (A⁻); G(T): 65,2%; G(NMR): 65%

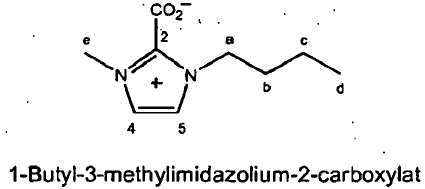

Summenformel: C₉H₁₄N₂O₂; Mᵣ = 182,1g/mol; ¹H-NMR (δ-werte, 400MHz, d⁶-DMSO): 0,879 (3H, t, J_{d,c}=7,33Hz: Hd); 1.240 (2H, tq, J_{c,b}=7,58Hz, J_{c,d}=7.33Hz: H_{c}); 1,707 (2H, tt, J_{b,c=}7,58Hz, J_{b,a}=7,33Hz: H_{b}); 3,945 (3H, s: Hₑ); 4,447 (2H, t, J_{a,b}=7,33Hz: Hₐ); 7,553 (1H, d, J_{4,5}=1,77Hz, B-Teil eines AB-Systems: H₄*); 7,610 (1H, d, J_{5,4}=1,77Hz, A-Teil eines AB-Systems: H₅*); *Zuordnung vertauschbar ; ESI-MS (+ mode, m/z): 182.1 (M*); G(T): nicht titrierbar, da nicht pH-aktiv

### Stufe 2 - Hydrolyse zum 1-Butyl-3-methylimidazolium-Hydrogencarbonat:

Nachdem das Reaktionsgemisch aus Stufe 1, welches die beiden Substanzen (Ie) und (IIe) enthält, auf ca. 80°C abgekühlt war und ein Großteil des Methanols entfernt worden war, wurde 1 Liter Wasser zugegeben (5,7 equiv.) Es wurde nun bei 80°C über einen Zeitraum von 12 Stunden gerührt. Nach dem Abkühlen wurden 2985g einer wäßrigen, schwach gelbgefärbte Lösung mit einem Gehalt von 63,8% BMIM-HCO₃ erhalten (98,5% der Theorie). Eine vom Wasser *in vacuo* bereite Probe wurde zur Charakterisierung herangezogen:

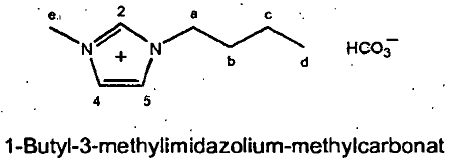

Summenformel: C₉H₁₆N₂O₃; Mᵣ = 200,2g/mol; ¹H-NMR (δ-Werte, 400MHz, d⁶-DMSO): 0,902 (3H, t, J_{d,c}=7,33Hz: H_{d}); 1,246 (2H, tq, J_{c,b}=7,58Hz. J_{c,d}=7,33Hz: H_{c}); 1,766 (2H, tt, J_{b,c}=7,58Hz, J_{b,a}=7,33Hz: H_{b}); 3,858 (3H, s: Hₑ); 4,171 (2H, t, J_{a,b}=7,33Hz: Hₐ); 7,716 (1H, t, J_{4,5}= J_{4,2}=J1,77Hz, B-Teil eines ABX-Systems: H₄*); 7,787 (1H, t, J_{5,4}=J_{5,2}=1,77Hz, A-Teil eines ABX-Systems: H₅*); 9.292 (1H, t, J_{2,4}=J_{2.5}=1,77Hz, X-Teil eines ABX-Systems: H₂); HCO₃⁻: nicht beobachtbar;* Zuordnung vertauschbar; ESI-MS (+ mode, m/z): 139,1 (K⁺) ; (- mode, m/z): 61,0 (A⁻); G(T) der Reaktionsmischung: 63,8% (98,5% d. Th.); G(T) der isolierten Festsubstanz: 99,0% (isolierte Festsubstanz eingewogen und vor Titration erneut hydrolysiert).

## Patentansprüche

1. Verfahren zur Herstellung von 1,3-heteroaromatischen Hydrogencarbonaten der Formel IIIa und/oder 1,3-heteroaromatischen Carbonaten der Formel IVa,
**dadurch gekennzeichnet,**
**daß** 1,3-heteroaromatische 2-Carboxylate der Formel la oder Gemische von 1,3-heteroaromatischen 2-Carboxylaten mit 1,3-heteroaromatischen Alkylcarbonaten und/oder 1,3-heteroaromatischen Arylcarbonaten der Formel IIa, mit Wasser zu 1,3-heteroaromatischen Hydrogencarbonaten und/oder 1,3-heteroaromatischen Carbonaten hydrolysiert werden,
- wobei diese Hydrolyse in Abwesenheit von Brønsted-Säuren oder Brønsted-Basen durchgeführt wird,
- wobei die 1,3-heteroaromatischen 2-Carboxylate (la), die 1,3-heteroaromatischen Alkylcarbonate oder 1,3-heteroaromatischen Arylcarbonate (IIa), die 1,3-heteroaromatischen Hydrogencarbonate (IIIa) und die 1,3-heteroaromatischen Carbonate (IVa) allgemein folgende Strukturen besitzen: 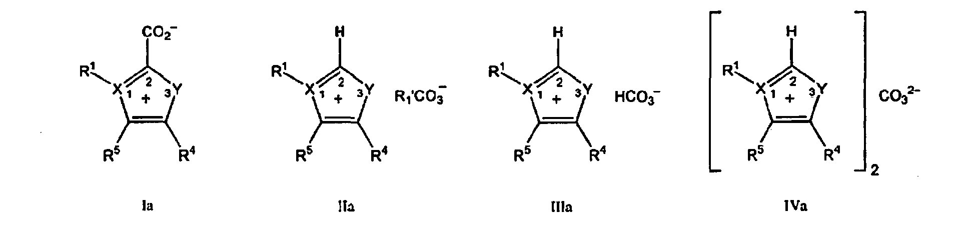
- wobei X Stickstoff (N) ist und Y Sauerstoff (O), Schwefel (S) oder eine N-R³, P-R³ Gruppe ist,
- wobei die Reste R¹,R^{1'} und R³ voneinander unabhängig lineare, cyclische, verzweigte, gesättigte oder ungesättigte Alkylreste, mono- oder polycyclische aromatische oder heteroaromatische Reste sind, und R³ zusätzlich Wasserstoff sein kann,
- wobei die Reste R⁴ und R⁵ voneinander unabhängig Wasserstoff, Halogen, Nitro, -SO₃H, -COOH, -OPO₂HR^{a}, -OPO₃R^{a}H, -OSO₃H sowie die Salze der fünf letztgenannten Reste, Sulfate, -OSO₃R^{a}, -SO₃R^{a}, -CO₂R^{a}, -OPO₂R^{a}R^{b}, -O-R^{a}, -SR^{a}, - NR^{a}R^{b}, -CONR^{a}R^{b}, -SO₂NR^{a}R^{b} oder gegebenenfalls substituierte lineare, cyclische, verzweigte, gesättigte oder ungesättigte Alkylreste, gegebenenfalls substituierte mono- oder polycyclische aromatische oder heteroaromatische Reste sind und R^{a} und R^{b} voneinander unabhängig für Wasserstoff, C₁-C₆-Alkyl, C₁-C₆-Halogenalkyl, Phenyl, Benzyl, Tolyl, Cyclopentyl oder Cyclohexyl stehen, oder
- wobei zwei benachbarte Reste der Reste R³,R⁴,R⁵ mit den Ringatomen, an welche sie gebunden sind, ein cyclisches oder aromatisches Ringsystem bilden, welche ggf. substituiert sein können oder
- im Falle der 1,3-heteroaromatischen Alkylcarbonate oder 1,3-heteroaromatischen Arylcarbonate (IIa) R¹ und R^{1'} gemeinsam eine -CHR^{c}-CHR^{d}-Kette, -CHR^{c}-CH2-CHR^{d}-Kette oder ggf. substuierten -C₆H₄-1,2-diyl-Rest bilden, wobei R^{c} und R^{d} unabhängig voneinander für H oder C₁-C₆-Alkyl stehen,
- wobei in den Verbindungen la, IIa, IIIa" IVa, voneinander unabhängig an Stelle eines Strukturelements, ausgewählt aus der Gruppe C-R⁴und C-R⁵, N stehen kann,
wobei
- R¹, R^{1'} voneinander unabhängig für C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} für Wasserstoff, C₁-C₆-Alkyl, C₁-C₆-Halogenalkyl, Phenyl, Benzyl, Tolyl, Cyclopentyl, Cyclohexyl, steht,
vorzugsweise C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl; CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
C₃- bis C₁₂-Cycloalkyl, insbesondere Cyclopentyl oder Cyclohexyl, C₃- bis C₁₂-Cycloalkyl, welches durch C₁-C₆-Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
CₙF_{2(n-a)-(1-b)}H_{2a-b}mit n ≤30, 0≤ a ≤ n und b = 0 oder 1;
C₂- bis C₃₀-Alkenyl, insbesondere 2-Propenyl, 3-Butenyl, cis-2-Butenyl, trans-2-Butenyl, oder CₙF_{2(n-a)-(1-b)}H_{2a-b} mit n ≤30, 0 ≤ a ≤ n und b = 0 oder 1;
C₃- bis C₁₂-Cycloalkenyl, insbesondere 3-Cyclopentenyl, 2-Cyclohexenyl, 3-Cyclohexenyl, 2,5-Cyclohexadienyl;
CₙF_{2(n-a)-(1-b)}H_{2a-b} mit n ≤30, 0 ≤ a ≤ n und b = 0 oder 1,
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
Aryl oder Heteroaryl mit 2 bis 30 Kohlenstoffatomen, welches durch ein bis drei C₁-C₆-Alkyl- oder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
oder C₆F₍₅₋ₐ₎Hₐ mit 0≤ a ≤ 5;
bedeuten,
- R³ Wasserstoff, sowie die unter R^{1'} genannten Reste, vorzugsweise Wasserstoff oder die unter R^{1'} vorzugsweise genannten Reste,
- R⁴, R⁵, für Wasserstoff, Halogen, OR^{a}, COR^{a}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl, Heteroaryl, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl, Cyclohexyl, steht;
vorzugsweise für Wasserstoff;
Halogen;
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
CₙF_{2(n-a)-(1-b})H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
OR^{a}, insbesondere Methoxy, Ethoxy, oder
COR^{a}, insbesondere Formyl oder Acetyl,
C₃- bis C₁₂-Cycloalkyl, insbesondere Cyclopentyl oder Cyclohexyl,
C₃- bis C₁₂-Cycloalkyl,welches durch C₁-C₆Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
CₙF_{2(n-a)-(1-b)}H_{2a-b}mit n ≤30, 0≤a≤n und b = 0 oder 1;
C₂- bis C₃₀-Alkenyl, insbesondere 2-Propenyl, 3-Butenyl, cis-2-Butenyl, trans-2-Butenyl, oder CₙF_{2(n-a)-(1-b)}H_{2a-b} mit n≤30, 0≤a≤n und b=0 oder 1;
C₃- bis C₁₂-Cycloalkenyl, insbesondere 3-Cyclopentenyl, 2-Cyclohexenyl, 3-Cyclohexenyl, 2,5-Cyclohexadienyl;
CₙF_{2(n-a)-(1-b)}H_{2a-b} mit n≤30, 0≤a≤n und b = 0 oder 1,
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
Aryl oder Heteroaryl mit 2 bis 30 Kohlenstoffatomen, welches durch ein bis drei C₁-C₆-Alkyl- opder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
oder C₆F₍₅₋ₐ₎Hₐ mit 0 ≤a≤ 5;
bedeuten; oder
- zwei benachbarte Reste der Reste R³,R⁴,R⁵ bilden mit den Ringatomen, an welchen sie gebunden sind, ein 5- gliedriges Ringsystem oder ein 5- gliedriges aromatisches Ringsystem, welche unsubstituiert sind, oder substituiert sind durch 1 bis 4 Reste aus der Gruppe C₁-C₆-Alkyl, Aryl, Heteroaryl, C₃-C₇-Cycloalkyl, Halogen, OR^{a}, SR^{a}, NR^{a}R^{b}, COOR^{a}, CO-NR^{a}R^{b} oder COR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl, Cyclohexyl, steht, oder
- im Falle der 1,3-heteroaromatischen Alkylcarbonate oder 1,3-heteroaromatischen Arylcarbonate (IIa) kann R¹ und R^{1'} gemeinsam eine -CHR^{c}-CHR^{d}-Kette, -CHR^{c}-CH₂-CHR^{d}-Kette oder ggf. substuierten -C₆H₄-1,2-diyl-Rest bilden, wobei R^{c} und R^{d} unabhängig voneinander für H oder C₁-C₆-Alkyl stehen.

2. Verfahren nach Anspruch 1, **dadurch gekennzeichnet, daß**
- R¹ R^{1'} voneinander unabhängig für C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} und R^{b} voneinander unabhängig für Wasserstoff, C₁-C₆-Alkyl, C₁-C₆-Halogenalkyl, Phenyl, Benzyl, Tolyl, Cyclopentyl und Cyclohexyl steht,
vorzugsweise C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
C₁-C₅-Alkyl, welches durch Phenyl, C₅-C₇-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
C₃- bis C₁₂-Cycloalkyl, insbesondere Cyclopentyl oder Cyclohexyl,
C₃- bis C₁₂-Cycloalkyl,welches durch C₁-C₆-Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
CₙF_{2(n-a)-(1-b)}H₂ₐ₋mit n ≤30, 0 ≤ a ≤ n und b = 0 oder 1;
C₂- bis C₃₀-Alkenyl, insbesondere 2-Propenyl, 3-Butenyl, cis-2-Butenyl, trans-2-Butenyl, oder CₙF₂₍ₙ₋ₐ₎-_{(1-b)}H_{2a-b} mit n ≤30, 0 ≤ a ≤ n und b = 0 oder 1;
C₃- bis C₁₂-Cycloalkenyl, insbesondere 3-Cyclopentenyl, 2-Cyclohexenyl, 3-Cyclohexenyl, 2,5-Cyclohexadienyl;
CₙF_{2(n-a)-(1-b)}H_{2a-b} mit n ≤30, 0 ≤ a ≤ n und b = 0 oder 1,
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
Aryl oder Heteroaryl mit 2 bis 30 Kohlenstoffatomen, welches durch ein bis drei C₁-C₆-Alkyl- oder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
oder C₆F₍₅₋ₐ₎Hₐ mit 0 ≤ a ≤ 5;
bedeuten,
- R³ Wasserstoff, sowie die unter R^{1'} genannten Reste, vorzugsweise Wasserstoff oder die unter R^{1'} vorzugsweise genannten Reste,
- R⁴, R⁵ Wasserstoff, Halogen, OR^{a}, COR^{a}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe Alkyl, Aryl, Heteroaryl, C₃-C₇-Cycloalkyl, Halogen, OR^{a}, SR^{a}, NR^{a}R^{b}, COOR^{a}, CO-NR^{a}R^{b}, COR^{a}, oder OR^{a}, wobei R^{a} für Wasserstoff, C₁-C₆-Alkyl, C₁-C₆-Halogenalkyl, Phenyl, Benzyl, Tolyl, Cyclopentyl und Cyclohexyl steht,
vorzugsweise für Wasserstoff;
Halogen;
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl;
- C₁-C₅-Alkyl, welches durch Phenyl, C₅-C7-Cycloalkyl substituiert sein kann, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁, C₁₂F₂₅; oder
OR^{a}, insbesondere Methoxy, Ethoxy, oder
COR^{a}, insbesondere Formyl oder Acetyl,
C₃- bis C₁₂-Cycloalkyl, insbesondere Cyclopentyl oder Cyclohexyl,
C₃- bis C₁₂-Cycloalkyl,weiches durch C₁-C₆Alkyl substituiert ist, insbesondere beispielsweise 2-Methyl-1-cyclopentyl, 3-Methyl-1-cyclopentyl, 2-Methyl-1-cyclohexyl, 3-Methyl-1-cyclohexyl, 4-Methyl-1-cyclohexyl;
CₙF_{2(n-a)-(1-b)}H_{2a-b} mit n ≤30, 0 ≤ a ≤ n und b = 0 oder 1;
C₂- bis C₃₀-Alkenyl, insbesondere 2-Propenyl, 3-Butenyl, cis-2-Butenyl, trans-2-Butenyl, oder CₙF₂₍ₙ₋ₐ₎₋₍₁-_{b)}H_{2a-b} mit n ≤30, 0 ≤ a ≤ n und b = 0 oder 1;
C₃- bis C₁₂-Cycloalkenyl, insbesondere 3-Cyclopentenyl, 2-Cyclohexenyl, 3-Cyclohexenyl, 2,5-Cyclohexadienyl;
CₙF_{2(n-a)-(1-b)}H_{2a-b} mit n ≤30, 0 ≤ a ≤ n und b = 0 oder 1,
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl;
Aryl oder Heteroaryl mit 2 bis 30 Kohlenstoffatomen, welches durch ein bis drei C₁-C₆-Alkyl- oder Phenyl-Reste substituiert ist, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenyl-phenyl;
oder C₆F₍₅₋ₐ₎Hₐ mit 0≤ a ≤ 5;
bedeuten; oder
- zwei benachbarte Reste der Reste R³,R⁴,R⁵ bilden mit den Ringatomen, an welchen sie gebunden sind, ein 5- gliedriges Ringsystem oder ein 5- gliedriges aromatisches Ringsystem, welche unsubstituiert sind, oder substituiert sind durch 1 bis 4 Reste aus der Gruppe C₁-C₆-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a},wobei R^{a} und R^{b} unabhängig voneinander für Wasserstoff, C₁-C₆ Alkyl, C₁-C₆-Halogenalkyl, Phenyl, Benzyl, Tolyl, Cyclopentyl und Cyclohexyl, stehen

3. Verfahren nach Anspruch 1 oder 2, **dadurch gekennzeichnet, daß** im Falle der 1,3-heteroaromatischen Alkylcarbonate oder 1,3-heteroaromatischen Arylcarbonate (IIa) R¹ und R^{1'} gemeinsam eine -CHR^{c}-CHR^{d}-Kette, -CHR^{c}-CH₂-CHR^{d}-Kette oder ggf. substuierten -C₆H₄-1,2-diyl-Rest bilden, wobei R^{c} und R^{d} unabhängig voneinander für H oder C₁-C₆-Alkyl stehen.

4. Verfahren nach einem der Ansprüche 1 bis 3, **dadurch gekennzeichnet, daß** das 1,3-heteroaromatische 2-Carboxylat la oder ein Gemisch aus 1,3-heteroaromatischem 2-Carboxylat und 1,3-heteroaromatischem Alkylcarbonat oder aus 1,3-heteroaromatischem 2-Carboxylat la und 1,3-heteroaromatischem Arylcarbonat IIa, mit einer Menge von 0,5 bis 20 Molequivalent Wasser, bezogen auf die Summe des 1,3 heteroaromatischen 2-Carboxylats und ggf. 1,3-heteroaromatischem Alkylcarbonats und/oder 1,3-heteroaromatischem Arylcarbonats, bevorzugt 2 bis 5 Molequivalent Wasser, besonders bevorzugt 3 Molequivalent Wasser, bei einer Temperatur von 30°C bis 350°C, bevorzugt 50°C bis 150°C, besonders bevorzugt 80°C bis 100°C, für eine Zeitdauer von 1 Minute bis 7 Tagen, bevorzugt 3 Stunden bis 2 Tagen, besonders bevorzugt 5 Stunden bis 12 Stunden, hydrolysiert wird.

5. Verfahren nach einem der Ansprüche 1 bis 4, **dadurch gekennzeichnet, daß** das 1,3-heteroaromatische 2-Carboxylat aus der Gruppe der folgenden Strukturen ausgewählt ist: 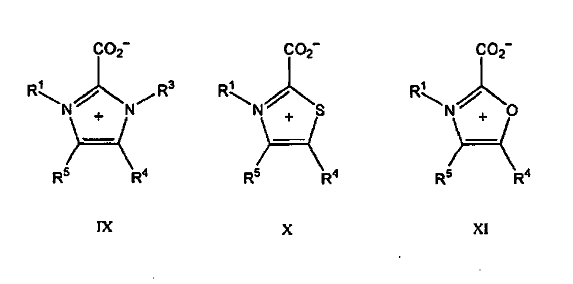 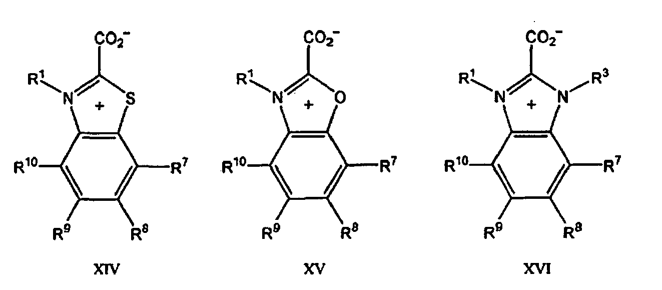 wobei die Reste R⁷ bis R¹⁰ folgende Bedeutung haben: Wasserstoff, C₁-C₆-Alkyl, Aryl, Heteroaryl, C₃-C₇-Cycloalkyl, Halogen, -OR^{a}, -SR^{a}, -NR^{a}R^{b}, -COOR^{a}, CO-NR^{a}R^{b} oder -COR^{a}, wobei R^{a} und R^{b} unabhängig voneinander für Wasserstoff, C₁-C₆-Alkyl, C₁-C₆-Halogenalkyl, Phenyl, Benzyl, Tolyl, Cyclopentyl und Cyclohexyl stehen und die Reste R¹, R⁴ bis R⁶ die voranstehenden Bedeutungen haben.

6. Verfahren nach einem der Ansprüche 1 bis 5, **dadurch gekennzeichnet, daß** das 1,3-heteroaromatische 2-Carboxylat la oder das Gemisch aus 1,3-heteroaromatischem 2-Carboxylat und 1,3-heteroaromatischem Alkylcarbonat IIa oder aus 1,3-heteroaromatischem 2-Carboxylat und 1,3-heteroaromatischem Arylcarbonat IIa durch Umsetzung eines 1,3-heterocyclischen Aromaten mit einem Dialkylcarbonat oder Diarylcarbonat oder Alkylarylcarbonat R¹OCOOR^{1'} entsteht,
- wobei der 1,3-heterocyclische Aromat: 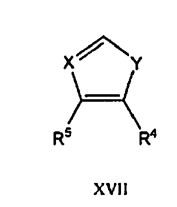 ist, wobei X, Y sowie die Reste R⁴ bis R⁵ die in den Ansprüchen 1 bis 6 angegebenen Bedeutungen haben.

7. Verfahren nach Anspruch 5, **dadurch gekennzeichnet, daß** der 1,3-heterocyclische Aromat ausgewählt ist aus der folgenden Gruppe: 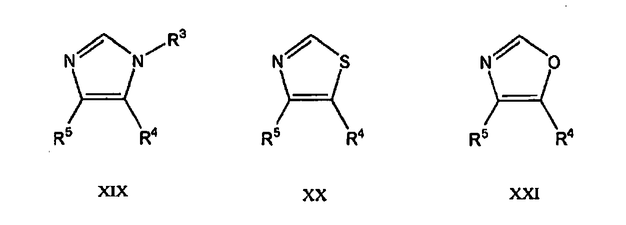 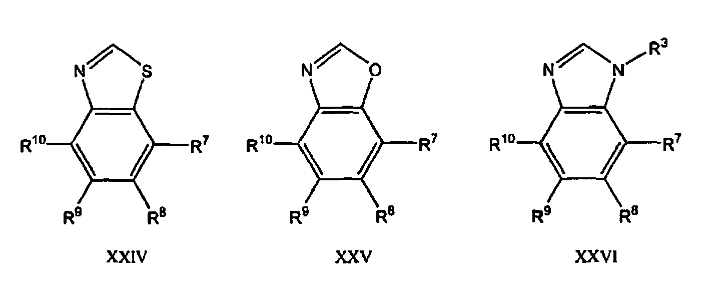 wobei die Reste R³ bis R¹⁰ die in den Ansprüchen 1 bis 5 genannten Bedeutungen haben.

8. Verfahren nach einem der Ansprüche 1 bis 7, **dadurch gekennzeichnet, daß** das Dialkylcarbonat oder Diarylcarbonat oder Alkylarylcarbonat R¹OCOOR¹ Ethylencarbonat, 1,2-Propylencarbonat oder 1,3-Propylencarbonat ist.

9. Verfahren nach einem der Ansprüche 1 bis 8, **dadurch gekennzeichnet, daß** die Hydrolyse des 1,3-heteroaromatischen 2-Carboxylats (la) oder eines Gemisches des 1,3-heteroaromatischen 2-Carboxylats mit einem 1,3-heteroaromatischen Alkylcarbonat und/oder 1,3-heteroaromatischen Arylcarbonat (IIa)
in Substanz oder in Gegenwart eines Hilfslösungsmittels, vorzugsweise eines Alkohols, insbesondere Methanol, Ethanol, Isopropanol, n-Propanol, n-Butanol, sec-Butanol, tert-Butanol, Pentanol, Hexanol, Heptanol, oder Octanol, eines Ether, eines Dialkylformamids, eines Ketons, eines Sulfoxids oder eines Nitrils, durchgeführt wird.

10. Verfahren nach einem der Ansprüche 7 bis 9, **dadurch gekennzeichnet, daß** die Hydrolyse des 1,3-heteroaromatischen 2-Carboxylats (la) oder eines Gemisches des 1,3-heteroaromatischen 2-Carboxylats mit einem 1,3-heteroaromatischen Alkylcarbonat und/oder 1,3-heteroaromatischen Arylcarbonat (IIa) in Substanz oder in Gegenwart eines Hilfslösungsmittels, vorzugsweise eines Alkohols, insbesondere Methanol, Ethanol, Isopropanol, n-Propanol, n-Butanol, sec-Butanol, tert-Butanol, Pentanol, Hexanol, Heptanol, Octanol oder Ether, Dialkylformamide, Ketone, Sulfoxide oder Nitrile, durchgeführt wird, und die Umsetzung des 1,3-heterocyclischen Aromaten (XVII, XIX bis XXI, XXIV bis XXVI) mit einem Dialkylcarbonat, Diarylcarbonat oder Alkylarylcarbonat R¹OCOOR^{1'} in Substanz oder in Gegenwart eines Hilfslösungsmittels, vorzugsweise eines Alkohols, insbesondere Methanol, Ethanol, Isopropanol, n-Propanol, n-Butanol, sec-Butanol, tert-Butanol, Pentanol, Hexanol, Heptanol, Octanol oder eines Ethers, eines Dialkylformamids, eines Ketons, eines Sulfoxids oder eines Nitrils, durchgeführt wird.

11. Verfahren nach einem der Ansprüche 1 bis 10, **dadurch gekennzeichnet, daß** der Hydrolyseprozeß batchweise, semikontinuierlich oder kontinuierlich erfolgt.

12. Verfahren nach einem der Ansprüche 1 bis 11, **dadurch gekennzeichnet, daß** der Hydrolyseprozeß unter Luft- oder Wasserdampfatmosphäre, oder unter Schutzgasatmosphäre, beispielsweise unter Stickstoff, Kohlendioxid oder Argon, durchgeführt wird.

13. Verfahren nach einem der Ansprüche 1 bis 12, **dadurch gekennzeichnet, daß** die Hydrolyse in Gegenwart eines Katalysators durchgeführt wird, wobei der Katalysator ein Element der 3. bis 13. Gruppe des Periodensystems oder der Gruppe der Lanthanoiden enthält.

14. Verfahren nach Anspruch 13, **dadurch gekennzeichnet, daß** der Katalysator ein Element ausgewählt aus der Gruppe Ruthenium, Osmium, Cobalt, Rhodium, Iridium, Nickel, Palladium, Platin, Titan, Zirkon, Vanadium, Mangan, Scandium, Chrom, Molybdän, Wolfram, Eisen, Kupfer, Silber, Aluminium, Lanthan, Cer, Neodym, Samarium, Gadolinium, Erbium und Lutetium enthält.

15. Verfahren nach einem der Ansprüche 1 bis 14, **dadurch gekennzeichnet, daß** die Reaktionsmischung gerührt, geschüttelt oder anderweitig durchmischt wird.

16. Verfahren nach einem der Ansprüche 1 bis 15, **dadurch gekennzeichnet, daß** die Umsetzung bei Druck von 1 bis 20 bar, vorzugsweise bei dem Eigendruck des Reaktionsgemisches, durchgeführt wird.

17. Verfahren nach einem der Ansprüche 1 bis 16, **dadurch gekennzeichnet, daß** nach beendeter Reaktion zur Aufarbeitung das Wasser teilweise und/oder das Hilfslösungsmittel gänzlich oder teilweise durch Destillation, Vakuumdestillation, Dünnschichtverdampfung, Kurzwegverdampfung, Rotations-verdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren entfernt wird.

## Claims

1. A method for manufacturing 1,3-heteroaromatic bicarbonates with formula IIIa and/or 1,3-heteroaromatic carbonates with formula IVa, **characterized in that**
1,3-heteroaromatic 2-carboxylates with formula Ia or mixtures of 1,3-heteroaromatic 2-carboxylates with 1,3-heteroaromatic alkyl carbonates and/or 1,3-heteroaromatic aryl carbonates with formula IIa are hydrolysed with water to 1,3-heteroaromatic bicarbonates and/or 1,3-heteroaromatic carbonates,
- wherein this hydrolysis is carried out in the absence of Brønsted acids or Brønsted bases,
- wherein the 1,3-heteroaromatic 2-carboxylates (Ia), the 1,3-heteroaromatic alkyl carbonates or 1,3-heteroaromatic aryl carbonates (IIa), the 1,3-heteroaromatic bicarbonates (IIIa) and the 1,3-heteroaromatic carbonates (IVa) have the following general structures: 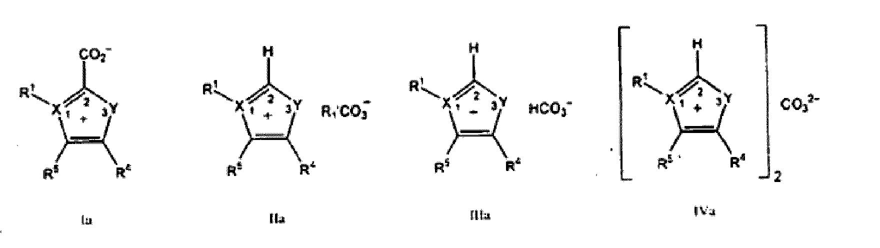
- wherein X is nitrogen (N) and Y is oxygen (0), sulphur (S) or a N-R³ or P-R³ group,
- wherein the residues R¹, R^{1'} and R³, independently of each other, are linear, cyclic, branched, saturated or unsaturated alkyl residues, mono- or poly-cyclic aromatic or heteroaromatic residues, and in addition, R³ may be hydrogen;
- wherein the residues R⁴ and R⁵, independently of each other, represent hydrogen, halogen, nitro, -SO₃H, -COOH, -OPO₂HR^{a}, -OPO₃R^{a}H, -OSO₃H and the salts of the latter five residues, sulphates, -OSO₃R^{a}, -SO₃R^{a}, -CO₂R^{a}, -OPO₂R^{a}R^{b}, -O-R^{a}, -SR^{a}, -NR^{a}R^{b}, -CONR^{a}R^{b} or -SO₂NR^{a}R^{b} or linear, cyclic, branched, saturated or unsaturated alkyl residues which may be substituted, mono- or poly-cyclic aromatic or heteroaromatic residues which may be substituted, and R^{a} and R^{b}, independently of each other, represent hydrogen, C₁-C₆ alkyl,
C₁-C₆ haloalkyl, phenyl, benzyl, tolyl, cyclopentyl or cyclohexyl, or
- wherein two adjacent residues of the residues R³, R⁴, R⁵ together with the ring atoms to which they are bonded form a cyclic or aromatic ring system which may be substituted, or
- in the case of the 1,3-heteroaromatic alkyl carbonates or 1,3-heteroaromatic aryl carbonates (IIa) , R¹ and R^{1'} together form a -CHR^{c}-CHR^{d}- chain, a -CHR^{c}-CH₂-CHR^{d}- chain or a -C₆H₄-1,2-diyl residue which may be substituted, wherein R^{c} and R^{d}, independently of each other, represent H or C₁-C₆ alkyl;
- wherein in the compounds Ia, IIa, IIIa, IVa, independently of each other, N can replace a structural element selected from the group C-R⁴ and C-R⁵;
wherein
- R¹, R¹', independently of each other, represents C₁-C₃₀ alkyl, C₃-C₁₂ cycloalkyl, C₂-C₃₀ alkenyl, C₃-C₁₂ cycloalkenyl, aryl or heteroaryl, wherein the latter 6 residues may carry one or more halogen residues and/or 1 to 3 residues selected from the group alkyl, aryl, heteroaryl, C₃-C₇ cycloalkyl, halogen, OR^{a}, SR^{a}, NR^{a}R^{b}, COOR^{a}, CO-NR^{a}R^{b}, COR^{a} or OR^{a}, wherein R^{a} represents hydrogen, C₁-C₆ alkyl, C₁-C₆ haloalkyl, phenyl, benzyl, tolyl, cyclopentyl or cyclohexyl,
preferably C₁-C₃₀ alkyl, in particular methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl;
C₁-C₅ alkyl, which may be substituted with phenyl or C₅-C₇ cycloalkyl, in particular phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl, CₙF_{2(n-a)-(1-b)}H_{2a-b}, with n ≤ 30, 0 ≤ a ≤n and b = 0 or 1, in particular CF₃. C₂F₅, C₆F₁₃, C₈F₁₇, C₁₀F₂₁ or C₁₂F₂₅; or
C₃ to C₁₂ cycloalkyl, in particular cyclopentyl or cyclohexyl, C₃ to C₁₂ cycloalkyl substituted with C₁-C₆ alkyl, in particular 2-methyl-1-cyclopentyl, 3-methyl-1-cyclopentyl, 2-methyl-1-cyclohexyl, 3-methyl-1-cyclohexyl or 4-methyl-1-cyclohexyl, for example;
CₙF₂₍ₙ₋ₐ₎-H_{2a-b}, with n ≤ 30, 0 ≤ a ≤ n and b = 0 or 1;
C₂ to C₃₀ alkenyl, in particular 2-propenyl, 3-butenyl, cis-2-butenyl or trans-2-butenyl, or CₙF_{2(n-a)-(1-b)}H_{2a-b} with n ≤ 30, 0 ≤ a ≤ n and b = 0 or 1;
C₃ to C₁₂ cycloalkenyl, in particular 3-cyclopentenyl, 2-cyclohexenyl, 3-cyclohexenyl or 2,5-cyclohexadienyl;
CₙF_{2(n-a)-(1-b)}H_{2a-b} with n ≤ 30, 0 ≤ a ≤ n and b = 0 or 1;
aryl or heteroaryl containing 2 to 30 carbon atoms, in particular phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl containing 2 to 30 carbon atoms substituted with one to three C₁-C₆ alkyl or phenyl residues, in particular 2-methylphenyl (2-tolyl), 3-methylphenyl (3-tolyl), 4-methylphenyl, 2-ethylphenyl, 3-ethylphenyl, 4-ethylphenyl, 2,3-dimethyl phenyl, 2,4-dimethylphenyl, 2,5-dimethylphenyl, 2,6-dimethylphenyl, 3,4-dimethylphenyl, 3,5-dimethylphenyl or 4-phenylphenyl;
or C₆F₍₅₋ₐ₎Hₐ, with 0 ≤ a ≤ 5;
- R³ represents hydrogen, as well as the residues cited for R^{1'}, preferably hydrogen or the residues cited for R^{1'},
- R⁴, R⁵ represents hydrogen, halogen, OR^{a}, COR^{a}, C₁-C₃₀ alkyl, C₃-C₁₂ cycloalkyl, C₂-C₃₀ alkenyl, C₃-C₁₂ cycloalkenyl, aryl or heteroaryl, wherein the latter 6 residues may carry one or more halogen residues and/or 1 to 3 residues selected from the group alkyl, aryl, heteroaryl, C₃-C₇ cycloalkyl, halogen, OR^{a}, SR^{a}, NR^{a}R^{b}, COOR^{a}, CO-NR^{a}R^{b}, COR^{a} or OR^{a}, wherein R^{a} and R^{b}, independently of each other, represents hydrogen, C₁-C₆, alkyl, C₁-C₆ haloalkyl, phenyl, benzyl, tolyl, cyclopentyl or cyclohexyl;
preferably hydrogen;
halogen;
C₁-C₃₀ alkyl, in particular methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl;
C₁-C₅ alkyl, which may be substituted with phenyl or C₅-C₇ cycloalkyl, in particular phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl,
CₙF_{2(n-a)-(1-b)}H_{2a-b}, with n ≤ 30, 0 ≤ a ≤ n and b = 0 or 1, in particular CF₃. C₂F₅, C₆F₁₃, C₈-F₁₇, C₁₀F21 or C₁₂F₂₅; or
OR^{a}, in particular methoxy or ethoxy, or
COR^{a}, in particular formyl or acetyl,
C₃ to C₁₂ cycloalkyl, in particular cyclopentyl or cyclohexyl,
C₃ to C₁₂ cycloalkyl substituted with C₁-C₆ alkyl, in particular 2-methyl-1-cyclopentyl, 3-methyl-1-cyclopentyl, 2-methyl-1-cyclohexyl, 3-methyl-1-cyclohexyl or 4-methyl-1-cyclohexyl, for example;
CₙF_{2(n-a)-(1-b)}H_{2a-b}, with n ≤ 30, 0 ≤ a ≤ n and b = 0 or 1;
C₂ to C₃₀ alkenyl, in particular 2-propenyl, 3-butenyl, cis-2-butenyl or trans-2-butenyl, or CₙF₂₍ₙ₋ₐ₎-_{(1-b)}H_{2a-b} with n ≤ 30, 0 ≤ a ≤ n and b = 0 or 1;
C₃ to C₁₂ cycloalkenyl, in particular 3-cyclopentenyl, 2-cyclohexenyl, 3-cyclohexenyl or 2,5-cyclohexadienyl;
CₙF_{2(n-a)-(1-b)}H_{2a-b} with n ≤ 30, 0 ≤ a ≤ n and b = 0 or 1,
aryl or heteroaryl containing 2 to 30 carbon atoms, in particular phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl containing 2 to 30 carbon atoms substituted with one to three C₁-C₆ alkyl or phenyl residues, in particular 2-methylphenyl (2-tolyl), 3-methylphenyl (3-tolyl), 4-methylphenyl, 2-ethylphenyl, 3-ethylphenyl, 4-ethylphenyl, 2,3-dimethyl phenyl, 2,4-dimethylphenyl, 2,5-dimethylphenyl, 2,6-dimethylphenyl, 3,4-dimethylphenyl, 3,5-dimethylphenyl or 4-phenylphenyl;
or C₆F₍₅₋ₐ₎Hₐ, with 0 ≤ a ≤ 5;
or
- two adjacent residues of the residues R³, R⁴, R⁵ together with the ring atoms to which they are bonded form a 5-membered ring system or a 5-membered aromatic ring system, which are unsubstituted or substituted with 1 to 4 residues from the group C₁-C₆ alkyl, aryl, heteroaryl, C₃-C₇ cycloalkyl, halogen, OR^{a}, SR^{a}, NR^{a}R^{b}, COOR^{a}, CO-NR^{a}R^{b} or COR^{a}, wherein R^{a} and R^{b}, independently of each other, represents hydrogen, C₁- C₆ alkyl, C₁-C₆ haloalkyl, phenyl, benzyl, tolyl, cyclopentyl or cyclohexyl, or
- in the case of the 1,3-heteroaromatic alkyl carbonates or 1,3-heteroaromatic aryl carbonates (IIa) , R¹ and R^{1'} together form a - CHR^{c}-CHR^{d}- chain, a -CHR^{c}-CH₂-CHR^{d}- chain or a -C₆H₄-1,2-diyl residue which may be substituted, wherein R^{c} and R^{d}, independently of each other, represent H or C₁-C₆ alkyl.

2. The method according to claim 1, **characterized in that**
- R¹, R^{1'}, independently of each other, represent C₁-C₃₀ alkyl, C₃-C₁₂ cycloalkyl, C₂-C₃₀ alkenyl, C₃-C₁₂ cycloalkenyl, aryl or heteroaryl, wherein the latter 6 residues may carry one or more halogen residues and/or 1 to 3 residues selected from the group alkyl, aryl, heteroaryl, C₃-C₇ cycloalkyl, halogen, OR^{a}, SR^{a}, NR^{a}R^{b}, COOR^{a}, CO-NR^{a}R^{b}, COR^{a} or OR^{a}, wherein R^{a} and R^{b}, independently of each other, represents hydrogen, C₁-C₆ alkyl, C₁-C₆ haloalkyl, phenyl, benzyl, tolyl, cyclopentyl or cyclohexyl,
preferably C₁-C₃₀ alkyl, in particular methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl;
C₁-C₅ alkyl, which may be substituted with phenyl or C₅-C₇ cycloalkyl, in particular phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl,
CₙF_{2(n-a)-(1-b)}H_{2a-b}, with n ≤ 30, 0 ≤ a ≤ n and b = 0 or 1, in particular CF₃, C₂F₅, C₆F₁₃, C₈F_{17,} C₁₀F₂₁ or C₁₂F₂₅; or
C₃ to C₁₂ cycloalkyl, in particular cyclopentyl or cyclohexyl,
C₃ to C₁₂ cycloalkyl substituted with C₁-C₆ alkyl, in particular 2-methyl-1-cyclopentyl, 3-methyl-1-cyclopentyl, 2-methyl-1-cyclohexyl, 3-methyl-1-cyclohexyl or 4-methyl-1-cyclohexyl, for example;
CₙF_{2(n-a)-(1-b)}H_{2a-b} with n ≤ 30, 0 ≤ a ≤ n and b = 0 or 1;
C₂ to C₃₀ alkenyl, in particular 2-propenyl, 3-butenyl, cis-2-butenyl or trans-2-butenyl, or CₙF_{2(n-a)-(1-b)}H_{2a-b} with n ≤ 30, 0 ≤ a ≤ n and b = 0 or 1;
C₃ to C₁₂ cycloalkenyl, in particular 3-cyclopentenyl, 2-cyclohexenyl, 3-cyclohexenyl or 2,5-cyclohexadienyl;
CₙF_{2(n-a)-(1-b)}H_{2a-b} with n ≤ 30, 0 ≤ a ≤ n and b = 0 or 1,
aryl or heteroaryl containing 2 to 30 carbon atoms, in particular phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl containing 2 to 30 carbon atoms substituted with one to three C₁-C₆ alkyl or phenyl residues, in particular 2-methylphenyl (2-tolyl), 3-methylphenyl (3-tolyl), 4-methylphenyl, 2-ethylphenyl, 3-ethylphenyl, 4-ethylphenyl, 2,3-dimethyl phenyl, 2,4-dimethylphenyl, 2,5-dimethylphenyl, 2,6-dimethylphenyl, 3,4-dimethylphenyl, 3,5-dimethylphenyl or 4-phenylphenyl;
or C₆F₍₅₋ₐ₎Hₐ, with 0 ≤ a ≤ 5;
- R³ represents hydrogen as well as the residues cited under R^{1'}, preferably hydrogen or the residues cited under R^{1'},
- R⁴, R⁵ represent hydrogen, halogen, OR^{a}, COR^{a}, C₁-C₃₀ alkyl, C₃-C₁₂ cycloalkyl, C₂-C₃₀ alkenyl, C₃-C₁₂ cycloalkenyl, aryl or heteroaryl, wherein the latter 6 residues may carry one or more halogen residues and/or 1 to 3 residues selected from the group alkyl, aryl, heteroaryl, C₃-C₇ cycloalkyl, halogen, OR^{a}, SR^{a}, NR^{a}R^{b}, COOR^{a}, CO-NR^{a}R^{b}, COR^{a} or OR^{a}, wherein R^{a} represents hydrogen, C₁-C₆ alkyl, C₁-C₆ haloalkyl, phenyl, benzyl, tolyl, cyclopentyl or cyclohexyl;
preferably hydrogen;
halogen;
C₁-C₃₀ alkyl, in particular methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl;
- C₁-C₅ alkyl, which may be substituted with phenyl or C₅-C₇ cycloalkyl, in particular phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl,
CₙF_{2(n-a)-(1-b)}H_{2a-b}, with n ≤ 30, 0 ≤ a ≤ n and b = 0 or 1, in particular CF₃, C₂F₅, C₆F₁₃, C₈F₁₇, C₁₀F₂₁ or C₁₂F₂₅; or
OR^{a}, in particular methoxy, ethoxy, or
COR^{a}, in particular formyl or acetyl,
C₃ to C₁₂ cycloalkyl, in particular cyclopentyl or cyclohexyl,
C₃ to C₁₂ cycloalkyl substituted with C₁-C₆ alkyl, in particular 2-methyl-1-cyclopentyl, 3-methyl-1-cyclopentyl, 2-methyl-1-cyclohexyl, 3-methyl-1-cyclohexyl or 4-methyl-1-cyclohexyl, for example;
CₙF_{2(n-a)-(1-b)}H_{2a-b}. with n ≤ 30, 0 ≤ a ≤ n and b = 0 or 1;
C₂ to C₃₀ alkenyl, in particular 2-propenyl, 3-butenyl, cis-2-butenyl or trans-2-butenyl, or CₙF_{2(n-a)-(1-b)}H_{2a-b} with n ≤ 30, 0 ≤ a ≤ n and b = 0 or 1;
C₃ to C₁₂ cycloalkenyl, in particular 3-cyclopentenyl, 2-cyclohexenyl, 3-cyclohexenyl or 2,5-cyclohexadienyl;
CₙF_{2(n-a)-(1-b)}H_{2a-b} with n ≤ 30, 0 ≤ a ≤ n and b = 0 or 1,
aryl or heteroaryl containing 2 to 30 carbon atoms, in particular phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl containing 2 to 30 carbon atoms substituted with one to three C₁-C₆ alkyl or phenyl residues, in particular 2-methylphenyl (2-tolyl), 3-methylphenyl (3-tolyl), 4-methylphenyl, 2-ethylphenyl, 3-ethylphenyl, 4-ethylphenyl, 2,3-dimethyl phenyl, 2,4-dimethylphenyl, 2,5-dimethylphenyl, 2,6-dimethylphenyl, 3,4-dimethylphenyl, 3,5-dimethylphenyl or 4-phenylphenyl;
or C₆F₍₅₋ₐ₎Hₐ, with 0 ≤ a ≤ 5;
or
- two adjacent residues of the residues R³, R⁴, R⁵ together with the ring atoms to which they are bonded form a 5-membered ring system or a 5-membered aromatic ring system, which are unsubstituted or substituted with 1 to 4 residues from the group C₁-C₆ alkyl, aryl, heteroaryl, C₃-C₇ cycloalkyl, halogen, OR^{a}, SR^{a}, NR^{a}R^{b}, COOR^{a}, CO-NR^{a}R^{b} or COR^{a}, wherein R^{a} and R^{b}, independently of each other, represents hydrogen, C₁-C₆ alkyl, C₁-C₆ haloalkyl, phenyl, benzyl, tolyl, cyclopentyl or cyclohexyl.

3. The method according to claim 1 or 2, **characterized in that** in the case of the 1,3-heteroaromatic alkyl carbonates or 1,3-heteroaromatic aryl carbonates (IIa), R¹ and R^{1'} together form a -CHR^{c}-CHR^{d}- chain, a -CHR^{c}-CH₂-CHR^{d}- chain or a -C₆H₄-1,2-diyl residue which may be substituted, wherein R^{c} and R^{d}, independently of each other, represent H or C₁-C₆ alkyl.

4. The method according to any one of claims 1 to 3, **characterized in that** the 1,3-heteroaromatic 2-carboxylate Ia or a mixture of 1,3-heteroaromatic 2-carboxylate and 1,3-heteroaromatic alkyl carbonate IIa or of 1,3-heteroaromatic 2-carboxylate 1a and 1,3-heteroaromatic aryl carbonate IIa is hydrolysed with a quantity of 0.5 to 20 mole equivalent of water, with respect to the sum of the 1,3-heteroaromatic 2-carboxylate and, if appropriate, 1,3-heteroaromatic alkyl carbonate and/or 1,3-heteroaromatic aryl carbonate,
preferably 2 to 5 mole equivalent of water, particularly preferably 3 mole equivalent of water, at a temperature of 30°C to 350°C, preferably 50°C to 150°C, particularly preferably 80°C to 100°C, for a time period of 1 minute to 7 days, preferably 3 hours to 2 days, particularly preferably 5 hours to 12 hours.

5. The method according to any one of claims 1 to 4, **characterized in that** the 1,3-heteroaromatic 2-carboxylate is selected from the group of the following structures: 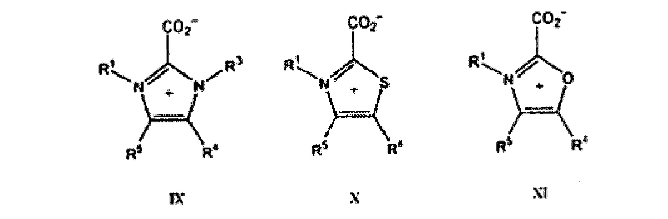 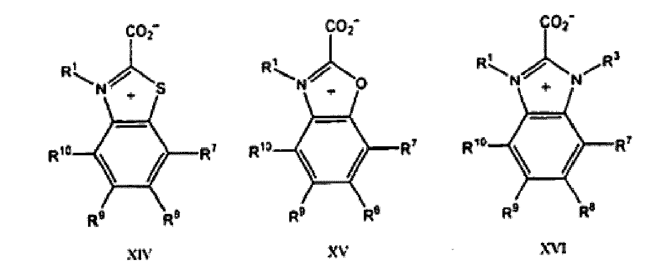 wherein the residues R⁷ to R¹⁰ represent the following: hydrogen, C₁-C₆ alkyl, aryl, heteroaryl, C₃-C₇ cycloalkyl, halogen, -OR^{a}, -SR^{a}, -NR^{a}R^{b}, -COOR^{a}, CO-NR^{a}R^{b} or -COR^{a}, wherein R^{a} and R^{b}, independently of each other, represent hydrogen,
C₁-C₆ alkyl, C₁-C₆ haloalkyl, phenyl, benzyl, tolyl, cyclopentyl or cyclohexyl, and the residues R¹, R⁴ to R⁶ have the meanings given above.

6. The method according to any one of claims 1 to 5, **characterized in that** the 1,3-heteroaromatic 2-carboxylate Ia or the mixture of 1,3-heteroaromatic 2-carboxylate and 1,3-heteroaromatic alkyl carbonate IIa or of 1,3-heteroaromatic 2-carboxylate and 1,3-heteroaromatic aryl carbonate IIa is produced by reacting a 1,3-heterocyclic aromatic compound with a dialkyl carbonate or diaryl carbonate or alkylaryl carbonate R¹OCOOR^{1'},
- wherein the 1,3-heterocyclic aromatic compound is: 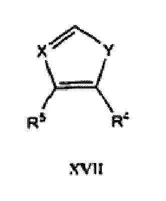 wherein X, Y and the residues R⁴ and R⁵ have the meanings given in claims 1 to 6.

7. The method according to claim 5, **characterized in that** the 1,3-heterocyclic aromatic compound is selected from the following group: 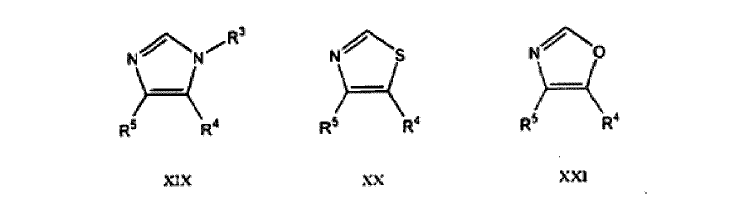 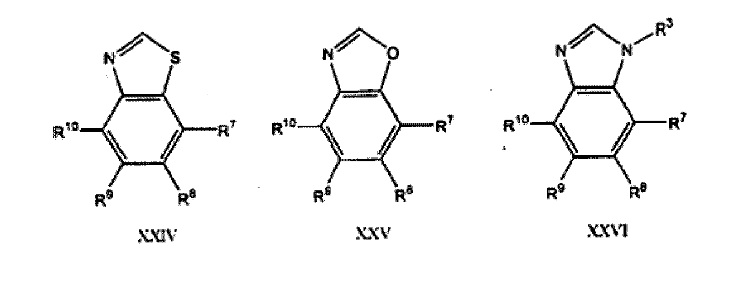 wherein the residues R³ to R¹⁰ have the meanings given in claims 1 to 5.

8. The method according to any one of claims 1 to 7, **characterized in that** the dialkyl carbonate or diaryl carbonate or alkylaryl carbonate R¹OCOOR^{1'} is ethylene carbonate, 1,2-propylene carbonate or 1,3-propylene carbonate.

9. The method according to any one of claims 1 to 8, **characterized in that** the hydrolysis of the 1,3-heteroaromatic 2-carboxylate (Ia) or a mixture of the 1,3-heteroaromatic 2-carboxylate with a 1,3-heteroaromatic alkyl carbonate and/or 1,3-heteroaromatic aryl carbonate (IIa) is carried out neat or in the presence of an auxiliary solvent, preferably an alcohol, in particular methanol, ethanol, isopropanol, n-propanol, n-butanol, sec-butanol, tert-butanol, pentanol, hexanol, heptanol or octanol, an ether, a dialkylformamide, a ketone, a sulphoxide or a nitrile.

10. The method according to any one of claims 7 to 9, **characterized in that** the hydrolysis of the 1,3-heteroaromatic 2-carboxylate (Ia) or a mixture of the 1,3-heteroaromatic 2-carboxylate with a 1,3-heteroaromatic alkyl carbonate and/or 1,3-heteroaromatic aryl carbonate (IIa) is carried out neat or in the presence of an auxiliary solvent, preferably an alcohol, in particular methanol, ethanol, isopropanol, n-propanol, n-butanol, sec-butanol, tert-butanol, pentanol, hexanol, heptanol, octanol or ethers, dialkylformamides, ketones, sulphoxides or nitriles, and the reaction of the 1,3-heterocyclic aromatic compound (XVII, XIX to XXI, XXIV to XXVI) is carried out with a dialkyl carbonate, diaryl carbonate or alkylaryl carbonate R¹OCOOR^{1'} neat or in the presence of an auxiliary solvent, preferably an alcohol, in particular methanol, ethanol, isopropanol, n-propanol, n-butanol, sec-butanol, tert-butanol, pentanol, hexanol, heptanol, octanol or an ether, a dialkylformamide, a ketone, a sulphoxide or a nitrile.

11. The method according to any one of claims 1 to 10, **characterized in that** the hydrolysis process is carried out batchwise, semi-continuously or continuously.

12. The method according to any one of claims 1 to 11, **characterized in that** the hydrolysis process is carried out under an atmosphere of air of steam or under a protective gas atmosphere, for example under nitrogen, carbon dioxide or argon.

13. The method according to any one of claims 1 to 12, **characterized in that** the hydrolysis is carried out in the presence of a catalyst, wherein the catalyst contains an element from group 3 to 13 of the periodic classification of the elements, or from the lanthanoids group.

14. The method according to claim 13, **characterized in that** the catalyst is an element selected from the group ruthenium, osmium, cobalt, rhodium, iridium, nickel, palladium, platinum, titanium, zirconium, vanadium, manganese, scandium, chromium, molybdenum, tungsten, iron, copper, silver, aluminium, lanthanum, cerium, neodymium, samarium, gadolinium, erbium and lutetium.

15. The method according to any one of claims 1 to 14, **characterized in that** the reaction mixture is shaken or stirred or mixed in another manner.

16. The method according to any one of claims 1 to 15, **characterized in that** the reaction is carried out under a pressure of 1 to 20 bar, preferably under the autonomous pressure of the reaction mixture.

17. The method according to any one of claims 1 to 16, **characterized in that** when the reaction is complete, in order to work it up, the water is partially removed, and/or the auxiliary solvent is partially or completely removed, by distillation, vacuum distillation, thin layer evaporation, short path evaporation, rotary evaporation, spray drying, osmosis, pervaporation, stripping with gas or steam, freezing out, freeze drying, chemical or physical adsorption or other suitable methods.

## Revendications

1. Procédé de préparation de bicarbonates 1,3-hétéroaromatiques de formule IIIa et/ou de carbonates 1,3-hétéroaromatiques de formule IVa,
**caractérisé en ce que**
des 2-carboxylates 1,3-hétéroaromatiques de formule la ou des mélanges de 2-carboxylates 1,3-hétéroaromatiques avec des carbonates d'alkyle 1,3-hétéroaromatiques et/ou avec des carbonates d'aryle 1,3-hétéroaromatiques de IIa sont hydrolysés à l'aide d'eau pour obtenir des bicarbonates 1,3-hétéroaromatiques et/ou des carbonates 1,3-hétéroaromatiques,
- ladite hydrolyse étant réalisée en absence d'acides de Br∅nsted ou de bases de Brønsted,
- les 2-carboxylates 1,3-hétéroaromatiques (Ia), les carbonates d'alkyle 1,3-hétéroaromatiques ou carbonates d'aryle 1,3-hétéroaromatiques (IIa), les bicarbonates 1,3-hétéroaromatiques (IIIa) et les carbonates 1,3-hétéroaromatiques (IVa) ayant en général les structures suivantes : 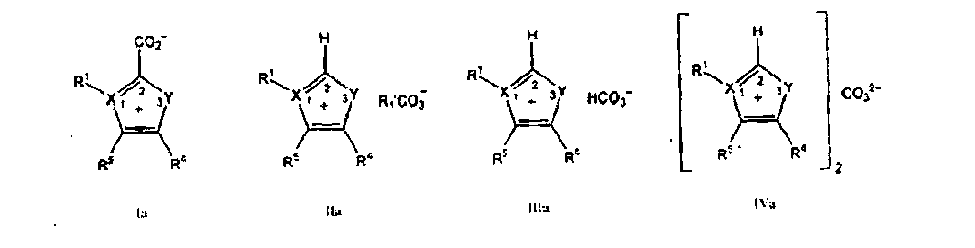
- X représentant l'azote (N) et Y représentant l'oxygène (O), le soufre (S) ou un groupe N-R³, P-R³,
- les radicaux R¹, R^{1'} et R³ représentant, indépendamment l'un de l'autre, des radicaux alkyle linéaires, cycliques, ramifiés, de nature saturée ou insaturée, des radicaux aromatiques ou hétéroaromatiques de nature mono- ou polycyclique, et R³ pouvant en outre représenter l'hydrogène,
- les radicaux R⁴ et R⁵ représentant, indépendamment l'un de l'autre, l'hydrogène, un halogène, nitro, -SO₃H, -COOH, -OPO₂HR^{a}, -OPO₃R^{a}H, -OSO₃H ainsi que les sels de ces cinq derniers radicaux, des sulfates, -OSO₃R^{a}, -SO₃R^{a}, -CO₂R^{a}, - OPO₂R^{a}R^{b}, -O-R^{a}, -SR^{a}, - NR^{a}R^{b}, -CONR^{a}R^{b}, -SO₂NR^{a}R^{b} ou, le cas échéant, des radicaux alkyle linéaires, cycliques, ramifiés, de nature saturée ou insaturée et le cas échéant substitués, des radicaux aromatiques ou hétéroaromatiques de nature mono- ou polycyclique, le cas échéant substitués, et R^{a} et R^{b} représentant, indépendamment l'un de l'autre, l'hydrogène, alkyle en C₁-C₆, alkyle halogéné en C₁-C₆, phényle, benzyle, tolyle, cyclopentyle ou cyclohexyle, ou
- deux radicaux voisins, parmi les radicaux R³, R⁴, R⁵, formant avec les atomes de l'anneau auxquels ils sont liés, un système annulaire de nature cyclique ou aromatique, des substituants pouvant y être présents le cas échéant, ou
- R¹ et R^{1'} formant ensemble, dans le cas des carbonates d'alkyle 1,3-hétéroaromatiques ou des carbonates d'aryle 1,3-hétéroaromatiques (IIa), une chaîne -CHR^{d}-CHR^{d}-, une chaîne -CHR^{c}-CH2-CHR^{d}- ou un radical -C₆H₄-1,2-diyle- le cas échéant substitué, R^{c} et R^{d} représentant indépendamment l'un de l'autre H ou un alkyle en C₁-C₆,
- N pouvant remplacer, dans les composés Ia, Ia, IIIa, IVa et de manière indépendante, un élément de structure choisi dans le groupe formé par C-R⁴ et C-R⁵,
dans lequel
- R¹, R^{1'} signifiant, indépendamment l'un de l' autre, un alkyle en C₁-C₃₀, un cycloalkyle en C₃-C₁₂, un alcényle en C₂-C₃₀, un cycloalcényle en C₃-C₁₂, un aryle ou un hétéroaryle, les 6 derniers radicaux cités pouvant porter un ou plusieurs radicaux halogènes et/ou 1 à 3 radicaux choisis dans le groupe formé par alkyle, aryle, hétéroaryle, cycloalkyle en C₃-C₇, halogène, OR^{a}, SR^{a}, NR^{a}R^{b}, COOR^{a}, CO-NR^{a}R^{b}, COR^{a} ou OR^{a}, R^{a} représentant l'hydrogène, un alkyle en C₁-C₆, un alkyle halogéné en C₁-C₆, phényle, benzyle, tolyle, cyclopentyle, cyclohexyle,
de préférence un alkyle en C₁-C₃₀, s'agissant notamment de méthyle, éthyle, 1-propyle, 2-propyle, 1-butyle, 2-butyle, 2-méthyl-1-propyle (isobutyle), 2-méthyl-2-propyle (tert.-butyle), 1-pentyle, 2-pentyle, 3-pentyle, 2-méthyl-1-butyle, 3-méthyl-1-butyle, 2-méthyl-2-butyle, 3-méthyl-2-butyle, 2,2-diméthyl-1-propyle, 1-hexyle, 2-hexyle, 3-hexyle, 2-méthyl-1-pentyle, 3-méthyl-1-pentyle, 4-méthyl-1-pentyle, 2-méthyl-2-pentyle, 3-méthyl-2-pentyle, 4-méthyl-2-pentyle, 2-méthyl-3-pentyle, 3-méthyl-3-pentyle, 2,2-diméthyl-1-butyle, 2,3-diméthyl-1-butyle, 3,3-diméthyl-1-butyle, 2-Ethyl-1-butyle, 2,3-diméthyl-2-butyle, 3,3-diméthyl-2-butyle,
heptyle, octyle, nonyle, décyle, undécyle, dodécyle, tridécyle, tétradécyle, pentadécyle, hexadécyle, heptadécyle, octadécyle, nonadécyle, icosyle, hénicosyle, docosyle, tricosyle, tétracosyle, pentacosyle, hexacosyle, heptacosyle, octacosyle, nonacosyle, triacontyle ;
un alkyle en C₁-C₅ pouvant être pourvu de substituants de type phényle, cycloalkyle en C₅-C₇, s'agissant notamment de phénylméthyle (benzyle), diphénylméthyle, triphénylméthyle, 2-phenyléthyle, 3-phénylpropyle, cyclopentylméthyle, 2-cyclopentyléthyle, 3-cyclopentylpropyle, cyclohexylméthyle, 2-cyclohexyléthyle, 3-cyclohexylpropyle ; CₙF_{2(n·a)·(1·b)}H_{2a·b} avec n ≤ 30, 0≤a≤n≤et b=0 ou 1, s' agissant notamment de CF₃, C₂F₅, C₆F₁₃, C₈F₁₇, C₁₀F₂₁, C₁₂F₂₅ ; ou
un cycloalkyle en C₃ à C₁₂, notamment le cyclopentyle ou le cyclohexyle ; un cycloalkyle en C₃ à C₁₂ pourvu de substituants de type alkyle en C₁-C₆, s'agissant notamment de 2-méthyl-1-cyclopentyle, 3-méthyl-1-cyclopentyle, 2-méthyl-1-cyclohexyle, 3-méthyl-1-cyclohexyle, 4-méthyl-1-cyclohexyle ;
CₙF₂(_{n·a)·(1·b)} H_{2a·b} avec n ≤ 30, 0 ≤ a ≤ n et b = 0 ou 1 ;
un alcényle en C₂ à C₃₀, s'agissant notamment de 2-propényle, 3-butényle, cis-2-butényle, trans-2-butényle, ou CₙF_{2(n·a) · (1·b)} H_{2a·b} avec n ≤ 30, 0 ≤ a ≤ n et b = 0 ou 1 ;
un cycloalcényle en C₃ à C₁₂, s'agissant notamment de 3-cyclopentényle, 2-cyclohexényle, 3-cyclohexényle, 2,5-cyclohexadiényle ;
CₙF_{2(n·a)·(1·b)}H_{2a·b} avec n ≤ 30, 0 ≤ a ≤ n et b = 0 ou 1,
un aryle ou hétéroaryle renfermant 2 à 30 atomes de carbone, s'agissant notamment de phényle, 1-naphtyle, 2-naphtyle, 1-pyrrolyle, 2-pyrrolyle, 3-pyrrolyle, 2-pyridinyle, 3-pyridinyle, 4-pyridinyle ;
un aryle ou hétéroaryle qui renferme 2 à 30 atomes de carbone et qui est pourvu d'un à trois substituants de type radical d'alkyle en C₁-C₆ ou phényle, s'agissant notamment de 2-méthylphényle (2-tolyle), 3-méthylphényle (3-tolyle), 4-méthylphényle, 2-éthylphényle, 3-éthylphényle, 4-éthylphényle, 2,3-diméthylphényle, 2,4-diméthylphényle, 2,5-diméthylphényle, 2,6-diméthylphényle, 3,4-diméthylphényle, 3,5-diméthylphényle, 4-phénylphényle ;
ou C₆F₍₅₋ₐ₎ Hₐ avec 0 ≤ a ≤ 5 ;
- R¹ signifiant l'hydrogène ainsi que les radicaux cités pour R¹', de préférence l'hydrogène ou les radicaux cités préférentiellement pour R¹',
- R⁴, R⁵ signifiant l'hydrogène, un halogène, OR^{a}, COR^{a}, un alkyle en C₁-C₃₀, un cycloalkyle en C₃-C₁₂, un alcényle en C₂-C₃₀, un cycloalcényle en C₃-C₁₂, un aryle ou un hétéroaryle, les 6 derniers radicaux cités pouvant porter un ou plusieurs radicaux halogènes et/ou 1 à 3 radicaux choisis dans le groupe formé par alkyle, aryle, hétéroaryle, cycloalkyle en C₃-C₇, halogène, OR^{a}, SR^{a}, NR^{a}R^{b}, COOR^{a}, CO-NR^{a}R^{b}, COR^{a} ou OR^{a}, R^{a} et R^{b} représentant, indépendamment l'un de l'autre, l'hydrogène, un alkyle en C₁-C₆, un alkyle halogéné en C₁-C₆, phényle, benzyle, tolyle, cyclopentyle, cyclohexyle ;
de préférence l'hydrogène ;
un halogène ;
un alkyle en C₁ à C₃₀, s'agissant notamment de méthyle, éthyle, 1-propyle, 2-propyle, 1-butyle, 2-butyle, 2-méthyl-1-propyle (isobutyle), 2-méthyl-2-propyle (tert.-butyle), 1-pentyle, 2-pentyle, 3-pentyle, 2-méthyl-1-butyle, 3-méthyl-1-butyle, 2-méthyl-2-butyle, 3-méthyl-2-butyle, 2,2-diméthyl-1-propyle, 1-hexyle, 2-hexyle, 3-hexyle, 2-méthyl-1-pentyle, 3-méthyl-1-pentyle, 4-méthyl-1-pentyle, 2-méthyl-2-pentyle, 3-méthyl-2-pentyle, 4-méthyl-2-pentyle, 2-méthyl-3-pentyle, 3-méthyl-3-pentyle, 2,2-diméthyl-1-butyle, 2,3-diméthyl-1-butyle, 3,3-diméthyl-1-butyle, 2-Ethyl-1-butyle, 2,3-diméthyl-2-butyle, 3,3-diméthyl-2-butyle, heptyle, octyle, nonyle, décyle, undécyle, dodécyle, tridécyle, tétradécyle, pentadécyle, hexadécyle, heptadécyle, octadécyle, nonadécyle, icosyle, hénicosyle, docosyle, tricosyle, tétracosyle, pentacosyle, hexacosyle, heptacosyle, octacosyle, nonacosyle, triacontyle ;
un alkyle en C₁-C₅ pouvant présenter des substituants de type phényle, cycloalkyle en C₅-C₇, s'agissant notamment de phénylméthyle (benzyle), diphénylméthyle, triphénylméthyle, 2-phenyléthyle, 3-phénylpropyle, cyclopentylméthyle, 2-cyclopentyléthyle, 3-cyclopentylpropyle, cyclohexylméthyle, 2-cyclohexyléthyle, 3-cyclohexylpropyle ;
CₙF_{2(n·a)·(1·b)}H_{2a·b} avec n ≤ 30, 0 ≤ a ≤ n et b = 0 ou 1, s' agissant notamment de CF₃, C₂F₅, C₆F₁₃, C₈F₁₇, C₁₀F₂₁, C₁₂F₂₅ ; ou
OR^{a}, s'agissant notamment de méthoxy, éthoxy, ou COR^{a}, s'agissant notamment de formyle ou d'acétyle,
un cycloalkyle en C₃ à C₁₂, s' agissant notamment de cyclopentyle ou de cyclohexyle,
un cycloalkyle en C₃ à C₁₂, pourvu d'un substituant de type alkyle en C₁-C₆, s'agissant par exemple notamment de 2-méthyl-1-cyclopentyle, 3-méthyl-1-cyclopentyle, 2-méthyl-1-cyclohexyle, 3-méthyl-1-cyclohexyle, 4-méthyl-1-cyclohexyle ;
CₙF_{2(n·a) · (1·b)} H_{2a·b} avec n ≤ 30, 0 ≤ a ≤ n et b = 0 ou 1 ;
un alcényle en C₂ à C₃₀, s'agissant notamment de 2-propényle, 3-butényle, cis-2-butényle, trans-2-butényle, ou CₙF_{2(n·a)·(1}·_{b)}H_{2a·b} avec n ≤ 30, 0 ≤ a ≤ n et b = 0 ou 1 ;
un cycloalcényle en C₃ à C₁₂, s'agissant notamment de 3-cyclopentényle, 2-cyclohexényle, 3-cyclohexényle, 2,5-cyclohexadiényle ;
CₙF_{2(n·a)·(1·b)}H_{2a·b} avec n ≤ 30, 0 ≤ a ≤ n et b = 0 ou 1,
un aryle ou hétéroaryle renfermant 2 à 30 atomes de carbone, s'agissant notamment de phényle, 1-naphtyle, 2-naphtyle, 1-pyrrolyle, 2-pyrrolyle, 3-pyrrolyle, 2-pyridinyle, 3-pyridinyle, 4-pyridinyle ;
un aryle ou hétéroaryle qui renferme 2 à 30 atomes de carbone et qui est pourvu d'un à trois substituants de type radical d'alkyle en C₁-C₆ ou phényle, s'agissant notamment de 2-méthylphényle (2-tolyle), 3-méthylphényle (3-tolyle), 4-méthylphényle, 2-éthylphényle, 3-éthylphényle, 4-éthylphényle, 2,3-diméthylphényle, 2,4-diméthylphényle, 2,5-diméthylphényle, 2,6-diméthylphényle, 3,4-diméthylphényle, 3,5-diméthylphényle, 4-phénylphényle ;
ou C₆F₍₅₋ₐ₎ Hₐ avec 0 ≤ s ≤ 5 ;
ou
- deux radicaux voisins, parmi les radicaux R³, R⁴, R⁵, formant avec les atomes de l'anneau auxquels ils sont liés, un système annulaire à 5 chaînons ou un système annulaire à 5 chaînons de nature aromatique, lesquels sont dépourvus de substituants ou substitués avec 1 à 4 radicaux issus du groupe formé par alkyle en C₁-C₆, aryle, hétéroaryle, cycloalkyle en C₃-C₇, halogène, OR^{a}, SR^{a}, NR^{a}R^{b}, COOR^{a}, CO-NR^{a}R^{b} ou COR^{a}, R^{a} et R^{b} représentant, indépendamment l'un de l'autre, l'hydrogène, un alkyle en C₁-C₆, un alkyle halogéné en C₁-C₆, phényle, benzyle, tolyle, cyclopentyle, cyclohexyle, ou
- R¹ et R^{1'} formant ensemble, dans le cas des carbonates d'alkyle 1,3-hétéroaromatiques ou des carbonates d'aryle 1,3-hétéroaromatiques (IIa), une chaîne -CHR^{c}-CHR^{d}-, une chaîne -CHR^{c}-CH₂-CHR^{d}- ou un radical -C₆H₄-1,2-diyle- le cas échéant substitué, R^{c} et R^{d} représentant indépendamment l'un de l'autre H ou un alkyle en C₁-C₆.

2. Procédé selon la revendication 1, **caractérisé en ce que**
- R¹, R^{1'} signifient, indépendamment l'un de l' autre, un alkyle en C₁-C₃₀, un cycloalkyle en C₃-C₁₂, un alcényle en C₂-C₃₀, un cycloalcényle en C₃-C₁₂, un aryle ou un hétéroaryle, les 6 derniers radicaux cités pouvant porter un ou plusieurs radicaux halogènes et/ou 1 à 3 radicaux choisis dans le groupe formé par alkyle, aryle, hétéroaryle, cycloalkyle en C₃-C₇, halogène, OR^{a}, SR^{a}, NR^{a}R^{b}, COOR^{a}, CO-NR^{a}R^{b}, COR^{a} ou OR^{a}, R^{a} et R^{b} représentant l'hydrogène, un alkyle en C₁-C₆, un alkyle halogéné en C₁-C₆, phényle, benzyle, tolyle, cyclopentyle, cyclohexyl,
de préférence un alkyle en C₁-C₃₀, s'agissant notamment de méthyle, éthyle, 1-propyle, 2-propyle, 1-butyle, 2-butyle, 2-méthyl-1-propyle (isobutyle), 2-méthyl-2-propyle (tert.-butyle), 1-pentyle, 2-pentyle, 3-pentyle, 2-méthyl-1-butyle, 3-méthyl-1-butyle, 2-méthyl-2-butyle, 3-méthyl-2-butyle, 2,2-diméthyl-1-propyle, 1-hexyle, 2-hexyle, 3-hexyle, 2-méthyl-1-pentyle, 3-méthyl-1-pentyle, 4-méthyl-1-pentyle, 2-méthyl-2-pentyle, 3-méthyl-2-pentyle, 4-méthyl-2-pentyle, 2-méthyl-3-pentyle, 3-méthyl-3-pentyle, 2,2-diméthyl-1-butyle, 2,3-diméthyl-1-butyle, 3,3-diméthyl-1-butyle, 2-Ethyl-1-butyle, 2,3-diméthyl-2-butyle, 3,3-diméthyl-2-butyle, heptyle, octyle, nonyle, décyle, undécyle,
dodécyle, tridécyle, tétradécyle, pentadécyle, hexadécyle, heptadécyle, octadécyle, nonadécyle, icosyle, hénicosyle, docosyle, tricosyle, tétracosyle, pentacosyle, hexacosyle, heptacosyle, octacosyle, nonacosyle, triacontyle ;
un alkyle en C₁-C₅ pouvant être pourvu de substituants de type phényle, cycloalkyle en C₅-C₇, s'agissant notamment de phénylméthyle (benzyle), diphénylméthyle, triphénylméthyle, 2-phenyléthyle, 3-phénylpropyle, cyclopentylméthyle, 2-cyclopentyléthyle, 3-cyclopentylpropyle, cyclohexylméthyle, 2-cyclohexyléthyle, 3-cyclohexylpropyle ;
CₙF_{2(n·a)·(1·b)}H_{2a·b} avec n ≤ 30, 0 ≤ a ≤ n et b = 0 ou 1, s' agissant notamment de CF₃, C₂F₅, C₆F₁₃, C₈F₁₇, C₁₀F₂₁, C₁₂F₂₅ ; ou
un cycloalkyle en C₃ à C₁₂, s' agissant notamment de cyclopentyle ou de cyclohexyle,
un cycloalkyle en C₃ à C₁₂, pourvu d'un substituant de type alkyle en C₁-C₆, s'agissant par exemple notamment de 2-méthyl-1-cyclopentyle, 3-méthyl-1-cyclopentyle, 2-méthyl-1-cyclohexyle, 3-méthyl-1-cyclohexyle, 4-méthyl-1-cyclohexyle ;
CₙF_{2(n·a)·(1·b)}H_{2a·b} avec n ≤ 30, 0 ≤ a ≤ n et b = 0 ou 1 ;
un alcényle en C₂ à C₃₀, s'agissant notamment de 2-propényle, 3-butényle, cis-2-butényle, trans-2-butényle, ou CₙF_{2(n·a)·(1·b)}H_{2a·b} avec n ≤ 30, 0 ≤ a ≤ n et b = 0 ou 1 ;
un cycloalcényle en C₃ à C₁₂, s'agissant notamment de 3-cyclopentényle, 2-cyclohexényle, 3-cyclohexényle, 2,5-cyclohexadiényle ;
CₙF_{2(n·a)·(1·b)}H_{2a·b} avec n ≤ 30, 0 ≤ a ≤ n et b = 0 ou 1,
un aryle ou hétéroaryle renfermant 2 à 30 atomes de carbone, s'agissant notamment de phényle, 1-naphtyle, 2-naphtyle, 1-pyrrolyle, 2-pyrrolyle, 3-pyrrolyle, 2-pyridinyle, 3-pyridinyle, 4-pyridinyle ;
un aryle ou hétéroaryle qui renferme 2 à 30 atomes de carbone et qui est pourvu d'un à trois substituants de type radical d'alkyle en C₁-C₆ ou phényle, s'agissant notamment de 2-méthylphényle (2-tolyle), 3-méthylphényle (3-tolyle), 4-méthylphényle, 2-éthylphényle, 3-éthylphényle, 4-éthylphényle, 2,3-diméthylphényle, 2,4-diméthylphényle, 2,5-diméthylphényle, 2,6-diméthylphényle, 3,4-diméthylphényle, 3,5-diméthylphényle, 4-phénylphényle ;
ou C₆F₍₅₋ₐ₎ Hₐ avec 0 ≤ a ≤ 5 ;
- R³ signifie l'hydrogène ainsi que les radicaux cités pour R¹', de préférence l'hydrogène ou les radicaux cités préférentiellement pour R¹',
R⁴, R⁵ signifient l'hydogène, un halogène, OR^{a}, COR^{a}, un alkyle en C₁-C₃₀, un cycloalkyle en C₃-C₁₂, un alcényle en C₂-C₃₀, un cycloalcényle en C₃-C₁₂, un aryle ou un hétéroaryle, les 6 derniers radicaux cités pouvant porter un ou plusieurs radicaux halogènes et/ou 1 à 3 radicaux choisis dans le groupe formé par alkyle, aryle, hétéroaryle, cycloalkyle en C₃-C₇, halogène, OR^{a}, SR^{a}, NR^{a}R^{b}, COLOR^{a}, CO-NR^{a}R^{b}, COR^{a} ou OR^{a}, R^{a} représentant l'hydrogène, un alkyle en C₁-C₆, un alkyle halogéné en C₁-C₆, phényle, benzyle, tolyle, cyclopentyle, cyclohexyle,
de préférence l'hydrogène ;
un halogène ;
un alkyle en C₁ à C₃₀, s'agissant notamment de méthyle, éthyle, 1-propyle, 2-propyle, 1-butyle, 2-butyle, 2-méthyl-1-propyle (isobutyle), 2-méthyl-2-propyle (tert.-butyle), 1-pentyle, 2-pentyle, 3-pentyle, 2-méthyl-1-butyle, 3-méthyl-1-butyle, 2-méthyl-2-butyle, 3-méthyl-2-butyle, 2,2-diméthyl-1-propyle, 1-hexyle, 2-hexyle, 3-hexyle, 2-méthyl-1-pentyle, 3-méthyl-1-pentyle, 4-méthyl-1-pentyle, 2-méthyl-2-pentyle, 3-méthyl-2-pentyle, 4-méthyl-2-pentyle, 2-méthyl-3-pentyle, 3-méthyl-3-pentyle, 2,2-diméthyl-1-butyle, 2,3-diméthyl-1-butyle, 3,3-diméthyl-1-butyle, 2-Ethyl-1-butyle, 2,3-diméthyl-2-butyle, 3,3-diméthyl-2-butyle, heptyle, octyle, nonyle, décyle, undécyle, dodécyle, tridécyle, tétradécyle, pentadécyle, hexadécyle, heptadécyle, octadécyle, nonadécyle, icosyle, hénicosyle, docosyle, tricosyle, tétracosyle, pentacosyle, hexacosyle, heptacosyle, octacosyle, nonacosyle, triacontyle ;
- un alkyle en C₁-C₅ pouvant présenter des substituants de type phényle, cycloalkyle en C₅-C₇, s'agissant notamment de phénylméthyle (benzyle), diphénylméthyle, triphénylméthyle, 2-phenyléthyle, 3-phénylpropyle, cyclopentylméthyle, 2-cyclopentyléthyle, 3-cyclopentylpropyle, cyclohexylméthyle, 2-cyclohexyléthyle, 3-cyclohexylpropyle ;
CₙF_{2(n·a)·(1·b)}H_{2a·b} avec n ≤ 30, 0 ≤ a ≤ n et b = 0 ou 1, s' agissant notamment de CF₃, C₂F₅, C₆F₁₃, C₈F₁₇, C₁₀F₂₁, C₁₂F₂₅ ; ou
OR^{a}, s'agissant notamment de méthoxy, éthoxy, ou COR^{a}, s'agissant notamment de formyle ou d'acétyle,
un cycloalkyle en C₃ à C₁₂, s' agissant notamment de cyclopentyle ou de cyclohexyle,
un cycloalkyle en C₃ à C₁₂, pourvu d'un substituant de type alkyle en C₁-C₆, s'agissant par exemple notamment de 2-méthyl-1-cyclopentyle, 3-méthyl-1-cyclopentyle, 2-méthyl-1-cyclohexyle, 3-méthyl-1-cyclohexyle, 4-méthyl-1-cyclohexyle ;
CₙF_{2(n·a)·(1·b)}H_{2a·b} avec n ≤ 30, 0 ≤ a ≤ n et b = 0 ou 1 ;
un alcényle en C₂ à C₃₀, s'agissant notamment de 2-propényle, 3-butényle, cis-2-butényle, trans-2-butényle, ou CₙF_{2(n·a)·(1·b)}H_{2a·b} avec n ≤ 30, 0 ≤ a ≤ n et b = 0 ou 1 ;
un cycloalcényle en C₃ à C₁₂, s'agissant notamment de 3-cyclopentényle, 2-cyclohexényle, 3-cyclohexényle, 2,5-cyclohexadiényle ;
CₙF_{2(n·a)·(1·b)}H_{2a·b} avec n ≤ 30, 0 ≤ a ≤ n et b = 0 ou 1 ;
un aryle ou hétéroaryle renfermant 2 à 30 atomes de carbone, s'agissant notamment de phényle, 1-naphtyle, 2-naphtyle, 1-pyrrolyle, 2-pyrrolyle, 3-pyrrolyle, 2-pyridinyle, 3-pyridinyle, 4-pyridinyle ;
un aryle ou hétéroaryle qui renferme 2 à 30 atomes de carbone et qui est pourvu d'un à trois substituants de type radical d'alkyle en C₁-C₆ ou phényle, s'agissant notamment de 2-méthylphényle (2-tolyle), 3-méthylphényle (3-tolyle), 4-méthylphényle, 2-éthylphényle, 3-éthylphényle, 4-éthylphényle, 2,3-diméthylphényle, 2,4-diméthylphényle, 2,5-diméthylphényle, 2,6-diméthylphényle, 3,4-diméthylphényle, 3,5-diméthylphényle, 4-phénylphényle ;
ou C₆F₍₅₋ₐ₎ Hₐ avec 0 ≤ a ≤ 5 ;
ou
- deux radicaux voisins, parmi les radicaux R³, R⁴, R⁵, forment avec les atomes de l'anneau auxquels ils sont liés, un système annulaire à 5 chaînons ou un système annulaire à 5 chaînons de nature aromatique, lesquels sont dépourvus de substituants ou substitués avec 1 à 4 radicaux issus du groupe formé par alkyle en C₁-C₆, aryle, hétéroaryle, cycloalkyle en C₃-C₇, halogène, OR^{a}, SR^{a} , NR^{a}R^{b}, COOR^{a}, CO-NR^{a}R^{b} ou COR^{a}, R^{a} et R^{b} représentant, indépendamment l'un de l'autre, l'hydrogène, un alkyle en C₁-C₆, un alkyle halogéné en C₁-C₆, phényle, benzyle, tolyle, cyclopentyle, cyclohexyle,

3. Procédé selon la revendication 1 ou 2, **caractérisé en ce que** R¹ et R¹' forment ensemble, dans le cas des carbonates d'alkyle 1,3-hétéroaromatiques ou des carbonates d'aryle 1,3-hétéroaromatiques (IIa), une chaîne -CHR^{c}-CHR^{d}-, une chaîne -CHR^{c}-CH₂-CHR^{d}- ou un radical -C₆H₄-1,2-diyle- le cas échéant substitué, R^{c} et R^{d} représentant indépendamment l'un de l'autre H ou un alkyle en C₁-C₆.

4. Procédé selon l'une des revendications 1 à 3, **caractérisé en ce que** le 2-carboxylate 1,3-hétéroaromatique Ia, ou un mélange de 2-carboxylate 1,3-hétéroaromatique et de carbonate d'alkyle 1,3-hétéroaromatique IIa ou bien de 2-carboxylate 1,3-hétéroaromatique Ia et de carbonate d'aryle 1,3-hétéroaromatique IIa, est hydrolysé en mettant en ouvre une quantité d'eau comprise entre 0,5 et 20 équivalents molaires, par rapport à la somme du 2-carboxylate 1,3-hétéroaromatique et le cas échéant du carbonate d'alkyle 1,3-hétéroaromatique et/ou du carbonate d'aryle 1,3-hétéroaromatique, de préférence comprise entre 2 et 5 équivalents molaires d'eau, avec une préférence particulière égale à 3 équivalents molaires d'eau, à une température comprise entre 30 °C et 350 °C, de préférence entre 50 °C et 150 °C, avec une préférence particulière entre 80 °C et 100 °C, pendant une durée comprise entre 1 minute et 7 jours, de préférence entre 3 heures et 2 jours, avec une préférence particulière entre 5 heures et 12 heures.

5. Procédé selon l'une des revendications 1 à 4, **caractérisé en ce que** le 2-carboxylate 1,3-hétéroaromatique est choisi dans le groupe formé par les structures suivantes : 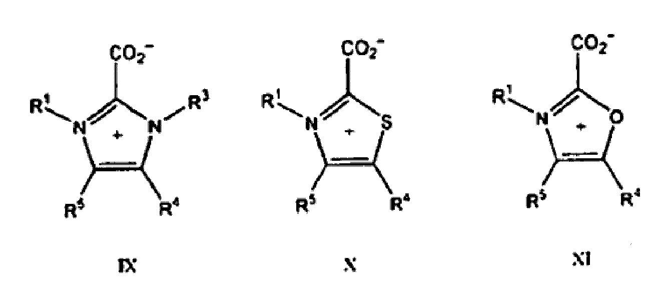 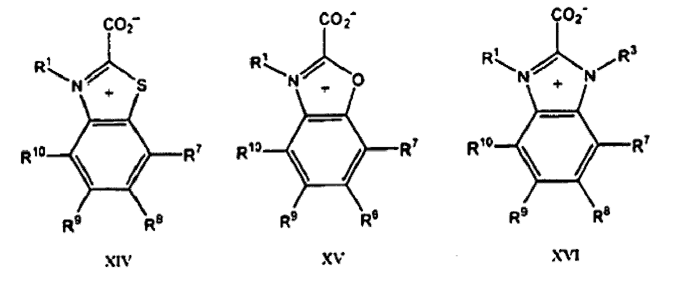 les radicaux R⁷ à R¹⁰ ayant la signification suivante : hydrogène, alkyle en C₁-C₆ aryle, hétéroaryle, cycloalkyle en C₃-C₇, halogène, -OR^{a}, -SR^{a}, -NR^{a}R^{b}, - COLOR^{a}, CO-NR^{a}R^{b} ou -COR^{a}, R^{a} et R^{b} représentant, indépendamment l'un de l'autre, l'hydrogène, un alkyle en C₁-C₆, un alkyle halogéné en C₁-C₆, phényle, benzyle, tolyle, cyclopentyle, cyclohexyle, et les radicaux R¹, R⁴ à R⁶ ayant les significations précédentes.

6. Procédé selon l'une des revendications 1 à 5, **caractérisé en ce que** le carboxylate 1,3-hétéroaromatique Ia ou le mélange de 2-carboxylate 1,3-hétéroaromatique et de carbonate d'alkyle 1,3-hétéroaromatique IIa ou bien de 2-carboxylate 1,3-hétéroaromatique et de carbonate d'aryle 1,3-hétéroaromatique IIa, est obtenu en faisant réagir un composé aromatique 1,3-hétérocyclique avec un carbonate de dialkyle ou carbonate de diaryle ou carbonate d' alkylaryle R¹OCOOR^{1'},
- le composé aromatique 1,3-hétérocyclique étant 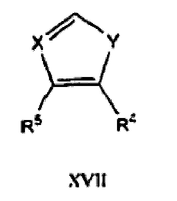 X, Y et Z ainsi que les radicaux R⁴ et R⁵ ayant les significations indiquées dans les revendications 1 à 6.

7. Procédé selon la revendication 5, **caractérisé en ce que** le composé aromatique 1,3-hétérocyclique est choisi dans le groupe suivant : 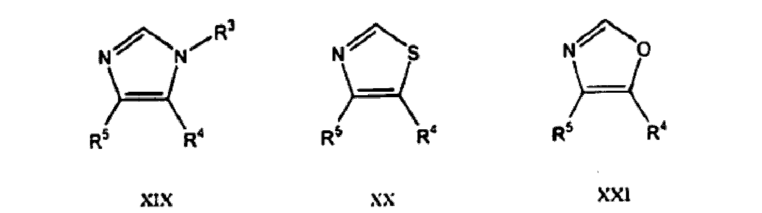 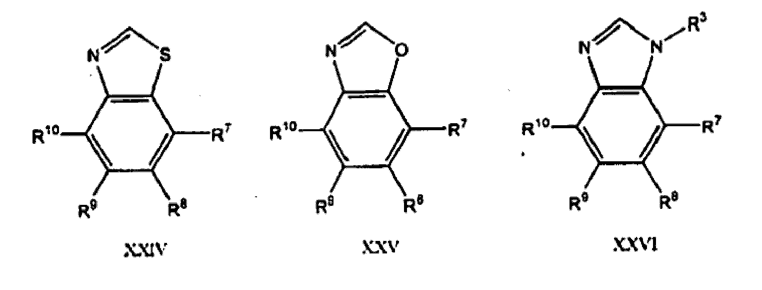 les radicaux R³ à R¹⁰ ayant les significations indiquées dans les revendications 1 à 5.

8. Procédé selon l'une des revendications 1 à 7, **caractérisé en ce que** le carbonate de dialkyle ou carbonate de diaryle ou carbonate d'alkylaryle R¹OCOOR^{1'} est le carbonate d'éthylène, le carbonate de 1,2-propylène ou le carbonate de 1,3-propylène.

9. Procédé selon l'une des revendications 1 à 8, **caractérisé en ce que** l'hydrolyse du carboxylate 1,3-hétéroaromatique (Ia), ou d'un mélange du 2-carboxylate 1,3-hétéroaromatique avec un carbonate d'alkyle 1,3-hétéroaromatique et/ou carbonate d'aryle 1,3-hétéroaromatique (IIa), est réalisée en masse ou en présence d'un solvant auxiliaire, s'agissant préférentiellement d'un alcool, notamment de méthanol, éthanol, isopropanol, n-propanol, n-butanol, sec.-butanol, tert.-butanol, pentanol, héxanol, heptanol ou octanol, d'un éther, d'un formamide de dialkyle, d'une cétone, d'un sulfoxyde ou d'un nitrile.

10. Procédé selon l'une des revendications 7 à 9, **caractérisé en ce que** l'hydrolyse du carboxylate 1,3-hétéroaromatique (Ia), ou d'un mélange du 2-carboxylate 1,3-hétéroaromatique avec un carbonate d'alkyle 1,3-hétéroaromatique et/ou carbonate d'aryle 1,3-hétéroaromatique (IIa), est réalisée en masse ou en présence d'un solvant auxiliaire, s'agissant préférentiellement d'un alcool, notamment de méthanol, éthanol, isopropanol, n-propanol, n-butanol, sec.-butanol, tert.-butanol, pentanol, héxanol, heptanol, octanol ou éthers, formamides de dialkyle, cétones, sulfoxydes ou nitriles, et la réaction du composé aromatique 1,3-hétérocyclique (XVII, XIX à XXI, XXIV à XXVI) avec un carbonate de dialkyle, carbonate de diaryle ou carbonate d'alkylaryle R¹OCOOR^{1'} est réalisée en masse ou en présence d'un solvant auxiliaire, s'agissant préférentiellement d'un alcool, notamment de méthanol, éthanol, isopropanol, n-propanol, n-butanol, sec.-butanol, tert-butanol, pentanol, héxanol, heptanol, octanol, ou d'un éther, d'un formamide de dialkyle, d'une cétone, d'un sulfoxyde ou d'un nitrile.

11. Procédé selon l'une des revendications 1 à 10, **caractérisé en ce que** le processus d'hydrolyse se déroule par lots, de manière semi-continue ou de manière continue.

12. Procédé selon l'une des revendications 1 à 11, **caractérisé en ce que** le processus d'hydrolyse est réalisé sous une atmosphère d'air ou de vapeur d'eau, ou bien sous atmosphère protectrice, par exemple sous azote, dioxyde de carbone ou argon.

13. Procédé selon l'une des revendications 1 à 12, **caractérisé en ce que** l'hydrolyse est réalisée en présence d'un catalyseur, ledit catalyseur contenant un élément issu du 3^{ème} au 13^{ème} groupe du tableau périodique ou du groupe des lanthanides.

14. Procédé selon la revendication 13, **caractérisé en ce que** ledit catalyseur contient un élément choisi dans le groupe formé par les éléments ruthénium, osmium, cobalt, rhodium, iridium, nickel, palladium, platine, titane, zirconium, vanadium, manganèse, scandium, chrome, molybdène, tungstène, fer, cuivre, argent, aluminium, lanthane, cérium, néodyme, samarium, gadolinium, erbium et lutécium.

15. Procédé selon l'une des revendications 1 à 14, **caractérisé en ce que** le mélange réactionnel est agité par un mouvement circulaire ou de va-et-vient ou qu'il est mélangé autrement.

16. Procédé selon l'une des revendications 1 à 15, **caractérisé en ce que** la réaction est réalisée sous une pression comprise entre 1 et 20 bar, s'agissant préférentiellement de pression générée par le mélange réactionnel même.

17. Procédé selon l'une des revendications 1 à 16, **caractérisé en ce qu'**une fois la réaction terminée, on procède à un traitement ultérieur consistant à éliminer partiellement l'eau et/ou à éliminer partiellement ou en totalité ledit solvant auxiliaire, en mettant en oeuvre une distillation, une distillation sous vide, une évaporation en couche mince, une évaporation à trajectoire courte, une évaporation rotative, une atomisation, une osmose, une pervaporation, un strippage à l'aide de gaz ou de vapeur d'eau, une solidification sélective, une lyophilisation, une adsorption chimique ou physique ou d'autres procédés appropriés.
